# EUROPEAN PATENT SPECIFICATION

(11) **EP 4 066 297 B1**
(45) Date of publication and mention of the grant of the patent: **24.12.2025**
(21) Application number: 20817346.8
(22) Date of filing: 26.11.2020
(51) Int. Cl.: C07D 471/04, C07D 519/00, H10K 85/60, H10K 50/165

(54) **ORGANIC ELECTRONIC DEVICE AND DISPLAY DEVICE COMPRISING THE ORGANIC ELECTRONIC DEVICE AS WELL AS ORGANIC COMPOUNDS FOR USE IN ORGANIC ELECTRONIC DEVICES**
ORGANISCHES ELEKTRONISCHES GERÄT UND ANZEIGEGERÄT DAMIT, UND ORGANISCHE VERBINDUNGEN FÜR ORGANISCHE ELEKTRONISCHE VORRICHTUNGEN
DISPOSITIF ÉLECTRONIQUE ORGANIQUE ET DISPOSITIFS D'ÉCLAIRAGE LE COMPRENANT, ET COMPOSÉS ORGANIQUE POUR DISPOSITIFS ELECTRONIQUES ORGANIQUES

(30) Priority: 27.11.2019 EP 19211912
(43) Date of publication of application: 05.10.2022
(73) Proprietor: Novaled GmbH, 01099 Dresden (DE)
(72) Inventor: GRÄF, Katja, 01099 Dresden (DE); KÖHLER, Martin, 01099 Dresden (DE); UVAROV, Vladimir, 01099 Dresden (DE); SENKOVVSKYY, Volodymyr, 01099 Dresden (DE); SCHOLZ, Johannes, 01099 Dresden (DE)
(74) Representative: Michalski Hüttermann & Partner Patentanwälte mbB
(86) International application number: PCT/EP2020/084457
(87) International publication number: WO 2021/105518

(56) References cited:
- KR-A- 20120 070 468
- VOLPI G ET AL: "Facile synthesis of novel blue light and large Stoke shift emitting tetradentate polyazines based on imidazo[1,5-a]pyridine", DYES AND PIGMENTS, ELSEVIER APPLIED SCIENCE PUBLISHERS. BARKING, GB, vol. 128, 18 December 2015 (2015-12-18), pages 96 - 100, XP029461104, ISSN: 0143-7208, DOI: 10.1016/J.DYEPIG.2015.12.005
- G. VOLPI ET AL: "Facile synthesis of novel blue light and large Stoke shift emitting tetradentate polyazines based on imidazo[1,5- a ]pyridine - Part 2", DYES AND PIGMENTS., vol. 143, 1 August 2017 (2017-08-01), GB, pages 284 - 290, XP055691437, ISSN: 0143-7208, DOI: 10.1016/j.dyepig.2017.04.034

## Description

### Technical Field

The present invention relates to an organic electronic device an display device comprising the organic electronic device. The invention further relates to novel compounds which can be of use in organic electronic devices.

### Background Art

Organic electronic devices, such as organic light-emitting diodes OLEDs, which are selfemitting devices, have a wide viewing angle, excellent contrast, quick response, high brightness, excellent operating voltage characteristics, and color reproduction. A typical OLED comprises an anode, a hole transport layer HTL, an emission layer EML, an electron transport layer ETL, and a cathode, which are sequentially stacked on a substrate. In this regard, the HTL, the EML, and the ETL are thin films formed from organic compounds.

When a voltage is applied to the anode and the cathode, holes injected from the anode move to the EML, via the HTL, and electrons injected from the cathode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has excellent efficiency and/or a long lifetime.

Performance of an organic light emitting diode may be affected by characteristics of the organic semiconductor layer, and among them, may be affected by characteristics of an organic material of the organic semiconductor layer.

Particularly, development of an organic semiconductor layer being capable of improving electron transport, electron injection and electron generation properties is needed. Thereby, operating voltage in OLEDs may be reduced. Lower operating voltage is important for reduced power consumption and improved battery life, esp. of mobile devices.

Further, development of an organic electronic device with improved efficiency is needed.

Increased efficiency is important for reducing power consumption and increasing battery life, for example of a mobile display device.

There remains a need to improve performance of organic semiconductor materials, organic semiconductor layers, as well as organic electronic devices thereof, in particular to achieve reduced operating voltage and increased efficiency through improving the characteristics of the compounds comprised therein. KR20120070468 (A) discloses an OLED devices comprising a compound with imidazo-pyridine structure.

### DISCLOSURE

An aspect of the present invention provides an organic electronic device according to the claims comprising an
anode, a cathode, at least one photoactive layer and an organic semiconductor layer, wherein the organic semiconductor layer is arranged between the at least one photoactive layer and the cathode; and wherein the organic semiconductor layer comprises a compound of Formula (1): 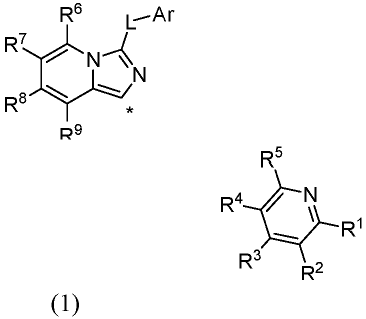 wherein
one of R¹ to R⁵ is a single bond to the 3-position (marked as "*") of the 2-azaindolizine moiety,
the further R¹ to R⁵ and R⁶ to R⁹ are independently selected from H, D, substituted or unsubstituted C₆ to C₁₈ aryl, substituted or unsubstituted C₃ to C₂₀ heteroaryl, C₁ to C₁₆ alkyl, C₁ to C₁₆ alkoxy, C₃ to C₁₆ branched alkyl, C₃ to C₁₆ cyclic alkyl, C₃ to C₁₆ branched alkoxy, C₃ to C₁₆ cyclic alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy, PX¹(R¹⁰)₂, F or CN, and/or wherein any two of adjacent R¹-R⁹ can be suitably substituted and linked together to form an unsubstituted or an C₆ to C₁₈ aryl-, C₃ to C₂₀ heteroaryl-, or C₁ to C₁₆ alkyl-substituted aromatic or heteroaromatic ring;
L is selected from a substituted or unsubstituted C₆ to C₂₄ arylene group or a substituted or unsubstituted C₂ to C₂₄ heteroarylene group;
Ar is selected from any of the following moieties D1 to D76: 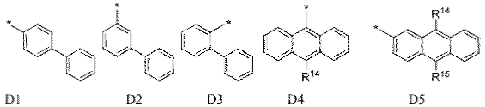 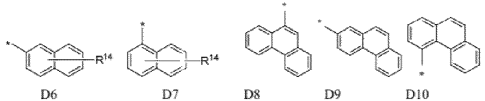 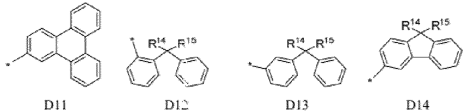 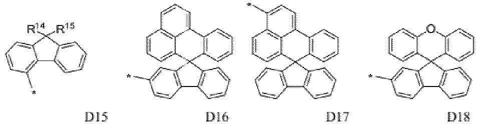 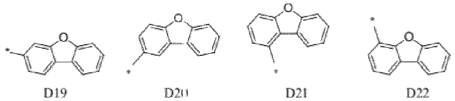 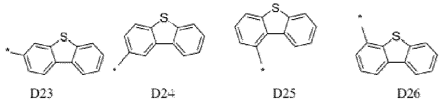 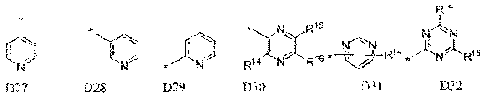 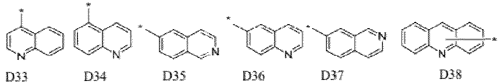 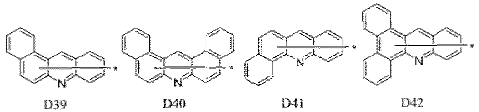 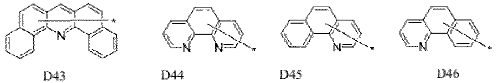 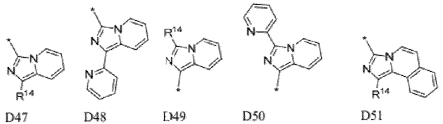 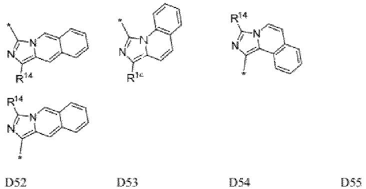 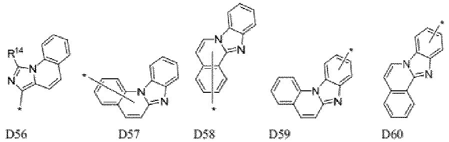 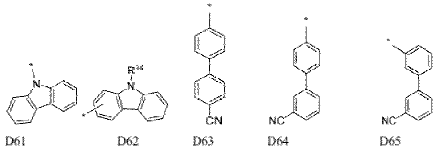 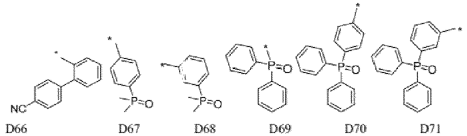 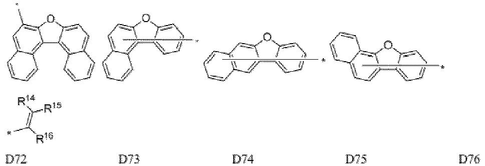
wherein R¹⁴, R¹⁵ and R¹⁶ are independently selected from H, C₁ to C₁₆ alkyl, C₁ to C₁₆ alkoxy, C₆ to C₁₈ aryl, C₃ to C₂₀ heteroaryl, perfluorinated C₁ to C₁₆ alkyl, perfluorinated C₁ to C₁₆ alkoxy, wherein R₁₄ and R₁₅ may be linked via a single bond or a heteroatom to form a ring;
wherein the substituents of L and Ar are independently selected from:
   H, D, C₆ to C₁₈ aryl, C₃ to C₂₀ heteroaryl, C₁ to C₁₆ alkyl, C₁ to C₁₆ alkoxy, C₃ to C₁₆ branched alkyl, C₃ to C₁₆ cyclic alkyl, C₃ to C₁₆ branched alkoxy, C₃ to C₁₆ cyclic alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy, F, CN or PX¹(R¹⁰)₂, wherein the substituents may be linked via a single bond or a heteroatom to form a ring,
   wherein R¹⁰ is independently selected from C₆ to C₁₂ aryl, C₃ to C₁₂ heteroaryl, C₁ to C₁₆ alkyl, C₁ to C₁₆ alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy;
   and X¹ is selected from O, S or Se, preferably O, and wherein the compound of formula (I) does not include the following compounds:

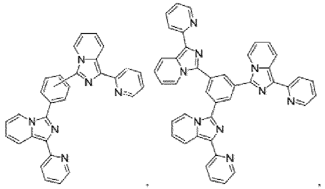

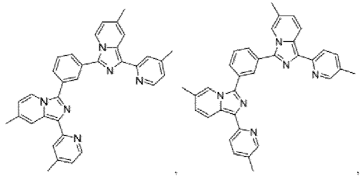

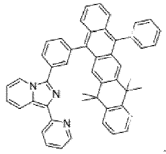

It should be noted that throughout the application and the claims any Rⁿ, Xⁿ, Ar or L always refer to the same moieties, unless otherwise noted.

In the present specification, when a definition is not otherwise provided, "substituted" refers to one substituted with a deuterium, C₁ to C₁₂ alkyl and C₁ to C₁₂ alkoxy.

However, in the present specification "aryl substituted" refers to a substitution with one or more aryl groups, which themselves may be substituted with one or more aryl and/or heteroaryl groups.

Correspondingly, in the present specification "heteroaryl substituted" refers to a substitution with one or more heteroaryl groups, which themselves may be substituted with one or more aryl and/or heteroaryl groups.

In the present specification, when a definition is not otherwise provided, an "alkyl group" refers to a saturated aliphatic hydrocarbyl group. The alkyl group may be a C₁ to C₁₂ alkyl group. More specifically, the alkyl group may be a C₁ to C₁₀ alkyl group or a C₁ to C₆ alkyl group. For example, a C₁ to C₄ alkyl group includes 1 to 4 carbons in alkyl chain, and may be selected from methyl, ethyl, propyl, iso-propyl, n-butyl, iso-butyl, sec-butyl, and tert-butyl.

Specific examples of the alkyl group may be a methyl group, an ethyl group, a propyl group, an isopropyl group, a butyl group, an iso-butyl group, a tert-butyl group, a pentyl group, a hexyl group.

The term "cycloalkyl" refers to saturated hydrocarbyl groups derived from a cycloalkane by formal abstraction of one hydrogen atom from a ring atom comprised in the corresponding cycloalkane. Examples of the cycloalkyl group may be a cyclopropyl group, a cyclobutyl group, a cyclopentyl group, a cyclohexyl group, a methylcyclohexyl group, an adamantly group and the like.

The term "hetero" is understood the way that at least one carbon atom, in a structure which may be formed by covalently bound carbon atoms, is replaced by another polyvalent atom. Preferably, the heteroatoms are selected from B, Si, N, P, O, S; more preferably from N, P, O, S.

In the present specification, "aryl group" refers to a hydrocarbyl group which can be created by formal abstraction of one hydrogen atom from an aromatic ring in the corresponding aromatic hydrocarbon. Aromatic hydrocarbon refers to a hydrocarbon which contains at least one aromatic ring or aromatic ring system. Aromatic ring or aromatic ring system refers to a planar ring or ring system of covalently bound carbon atoms, wherein the planar ring or ring system comprises a conjugated system of delocalized electrons fulfilling Hückel's rule. Examples of aryl groups include monocyclic groups like phenyl or tolyl, polycyclic groups which comprise more aromatic rings linked by single bonds, like biphenylyl, and polycyclic groups comprising fused rings, like naphtyl or fluoren-2-yl.

Analogously, under heteroaryl, it is especially where suitable understood a group derived by formal abstraction of one ring hydrogen from a heterocyclic aromatic ring in a compound comprising at least one such ring.

Under heterocycloalkyl, it is especially where suitable understood a group derived by formal abstraction of one ring hydrogen from a saturated cycloalkyl ring in a compound comprising at least one such ring.

The term "fused aryl rings" or "condensed aryl rings" is understood the way that two aryl rings are considered fused or condensed when they share at least two common sp²-hybridized carbon atoms

In the present specification, the single bond refers to a direct bond.

In the context of the present invention, "different" means that the compounds do not have an identical chemical structure.

The term "free of", "does not contain", "does not comprise" does not exclude impurities which may be present in the compounds prior to deposition. Impurities have no technical effect with respect to the object achieved by the present invention.

The term "contacting sandwiched" refers to an arrangement of three layers whereby the layer in the middle is in direct contact with the two adjacent layers.

The terms "light-absorbing layer" and "light absorption layer" are used synonymously.

The terms "light-emitting layer", "light emission layer" and "emission layer" are used synonymously.

The terms "OLED", "organic light-emitting diode" and "organic light-emitting device" are used synonymously.

In the specification, hole characteristics refer to an ability to donate an electron to form a hole when an electric field is applied and that a hole formed in the anode may be easily injected into the emission layer and transported in the emission layer due to conductive characteristics according to a highest occupied molecular orbital (HOMO) level.

In addition, electron characteristics refer to an ability to accept an electron when an electric field is applied and that electrons formed in the cathode may be easily injected into the emission layer and transported in the emission layer due to conductive characteristics according to a lowest unoccupied molecular orbital (LUMO) level.

### Advantageous Effects

Surprisingly, it was found that the organic electronic device according to the invention solves the problem underlying the present invention by enabling devices in various aspects superior over the organic electroluminescent devices known in the art, in particular with respect to operating voltage and efficiency.

According to one embodiment of the present invention, the substituents of the further R¹ to R⁵ and R⁶ to R⁹ are independently selected from: D, -CH=, C₆ to C₁₈ aryl, C₃ to C₂₀ heteroaryl, C₁ to C₁₆ alkyl, C₁ to C₁₆ alkoxy, C₃ to C₁₆ branched alkyl, C₃ to C₁₆ cyclic alkyl, C₃ to C₁₆ branched alkoxy, C₃ to C₁₆ cyclic alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy, PX¹(R¹⁰)₂, F or CN

According to one embodiment of the present invention, the compound of formula (1) has the following structure according to formula (1a):

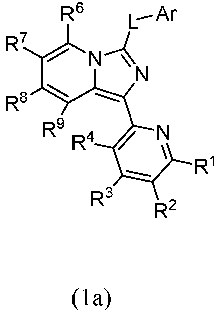

According to one embodiment of the present invention the organic layer and/or the compound of formula (1) and/or (1a) are non-emissive.

In the context of the present specification the term "essentially non-emissive" or "non-emissive" means that the contribution of the compound or layer to the visible emission spectrum from the device is less than 10 %, preferably less than 5 % relative to the visible emission spectrum. The visible emission spectrum is an emission spectrum with a wavelength of about ≥ 380 nm to about ≤ 780 nm.

According to one embodiment of the present invention the R¹ to R⁹ which do not form a single bond to the 3-position of the 2-azaindolizine moiety are independently selected from H, - CH=, C₁ to C₄ alkyl, F or CN.

According to one embodiment of the present invention the compound of formula (1) is selected from one of the following formulas (2a) to (2f) 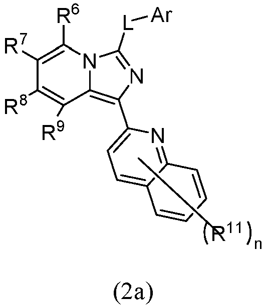 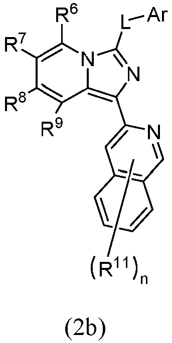 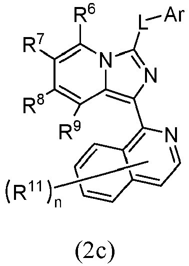 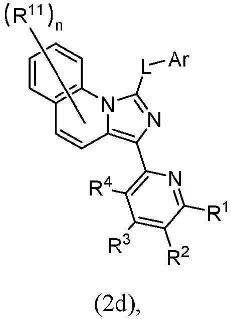 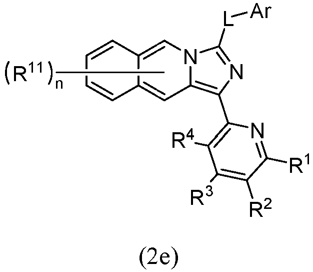 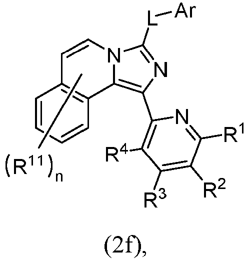 wherein R¹¹ is independently selected from D, C₆ to C₁₈ aryl, C₃ to C₂₀ heteroaryl, C₁ to C₁₆ alkyl, C₁ to C₁₆ alkoxy, C₃ to C₁₆ branched alkyl, C₃ to C₁₆ cyclic alkyl, C₃ to C₁₆ branched alkoxy, C₃ to C₁₆ cyclic alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy, PX¹(R¹⁰)₂, F or CN; and
n is an integer from 0 to 4.

According to one embodiment of the present invention, the moiety L comprises one or two ring systems connected via a single bond.

According to one embodiment of the present invention, the moiety L comprises one to three rings which may be fused or connected via a single bond.

According to one embodiment of the present invention, the moiety L is selected from an unsubstituted C₆ to C₂₄ arylene group or an unsubstituted C₂ to C₂₄ heteroarylene group.

According to one embodiment of the present invention, the moiety L is selected from a unsubstituted, alkyl- or aryl substituted C₆ to C₁₈ arylene group or a unsubstituted, alkyl- or aryl substituted C₃ to C₁₂ heteroarylene group.

According to one embodiment of the present invention, the moiety L is selected from a unsubstituted C₆ to C₁₈ arylene group or a unsubstituted C₃ to C₁₂ heteroarylene group.

According to one embodiment of the present invention, the moiety L is selected from a unsubstituted, alkyl- or aryl substituted C₆ to C₁₈ arylene group or a unsubstituted, alkyl- or aryl substituted C₃ to C₁₂ heteroarylene group comprising O or S atoms.

According to one embodiment of the present invention, the moiety L is selected from a unsubstituted, alkyl- or aryl substituted C₆ to C₁₈ arylene group or a unsubstituted, alkyl- or aryl substituted C₃ to C₁₂ heteroarylene group and is free of sp³-hybridised carbon atoms.

According to one embodiment of the present invention, the moiety L is selected from a unsubstituted, alkyl- or aryl substituted C₆ to C₁₈ arylene group or a unsubstituted, alkyl- or aryl substituted C₃ to C₁₂ heteroarylene group comprising O or S atoms and is free of sp³-hybridised carbon atoms.

According to one embodiment of the present invention, the moiety L is selected from a unsubstituted, alkyl- or aryl substituted C₆ to C₁₄ arylene group or a unsubstituted, alkyl- or aryl substituted C₃ to C₁₂ heteroarylene group.

According to one embodiment of the present invention the moiety L is selected any of the following moieties E1 to E26: 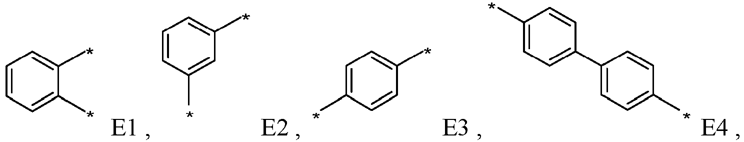 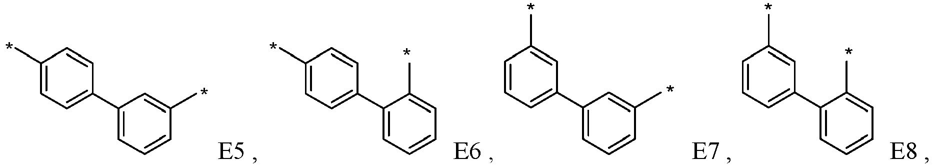 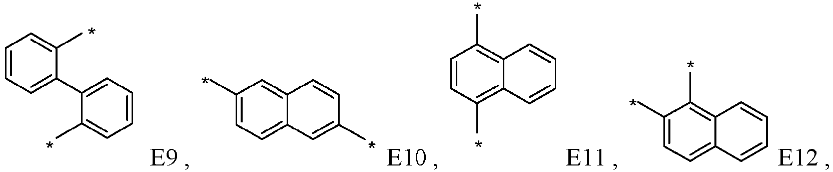 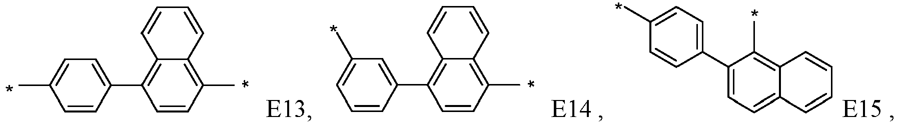 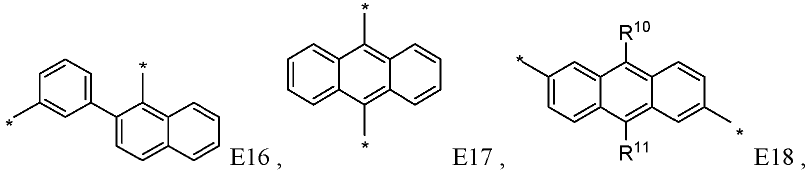 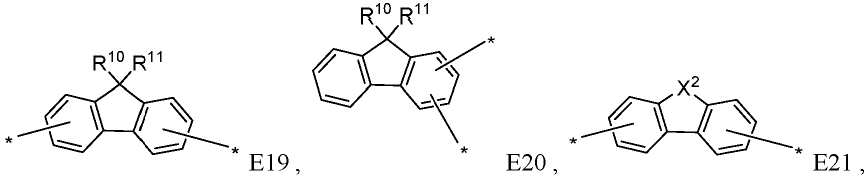 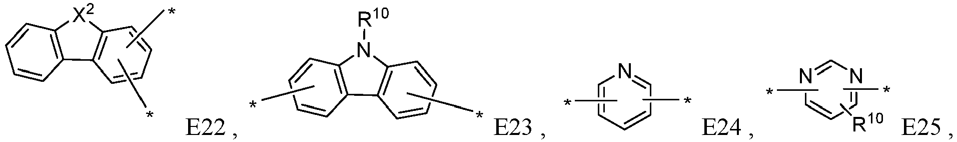 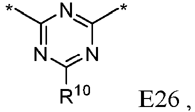 wherein
X² is selected from O or S, preferably O;
R¹⁰ and R¹¹ are independently selected from H, C₁ to C₁₆ alkyl, C₁ to C₁₆ alkoxy, C₆ to C₁₈ aryl, C₃ to C₂₀ heteroaryl, perfluorinated C₁ to C₁₆ alkyl, perfluorinated C₁ to C₁₆ alkoxy.

Especially preferred are moieties E1 to E18 and E21 to E22, alternatively E1 to E16 and E21 to E22, alternatively E1 to E16, alternatively E1, E2, E5 to E16 and E20.

According to one embodiment of the present invention, the moiety Ar is selected from the moieties D1 to D76.

According to one embodiment of the present invention, the substituents on Ar are independently selected from: H, D, C₆ to C₁₈ aryl, C₃ to C₂₀ heteroaryl, C₁ to C₁₆ alkyl, C₁ to C₁₆ alkoxy, C₃ to C₁₆ branched alkyl, C₃ to C₁₆ cyclic alkyl, C₃ to C₁₆ branched alkoxy, C₃ to C₁₆ cyclic alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy, F, CN, wherein the substituents may be linked via a single bond or a heteroatom to form a ring.

According to one embodiment of the present invention, the substituents of L and Ar are independently selected from: H, D, C₆ to C₁₈ aryl, C₃ to C₂₀ heteroaryl, C₁ to C₁₆ alkyl, C₁ to C₁₆ alkoxy, C₃ to C₁₆ branched alkyl, C₃ to C₁₆ cyclic alkyl, C₃ to C₁₆ branched alkoxy, C₃ to C₁₆ cyclic alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy, F or PX¹(R¹⁰)₂, wherein the substituents may be linked via a single bond or a heteroatom to form a ring.

According to one embodiment of the present invention, the moiety Ar is selected from any of the following moieties D1 to D76: 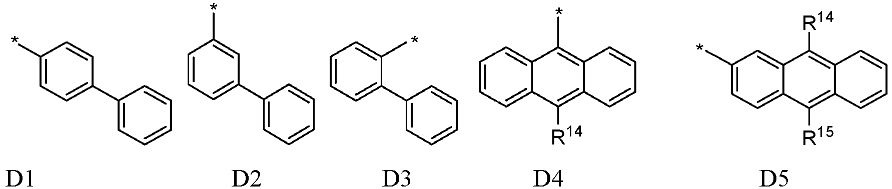 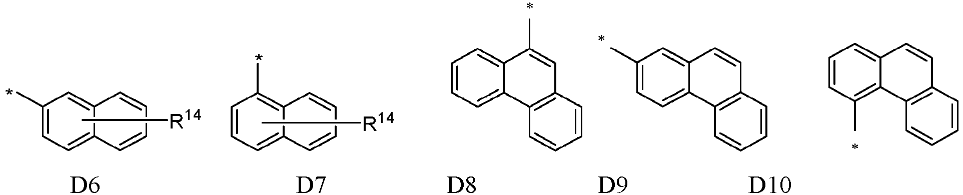 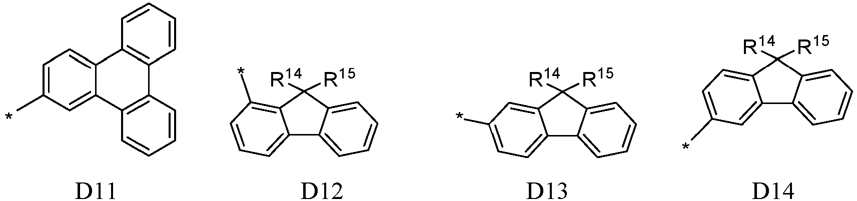 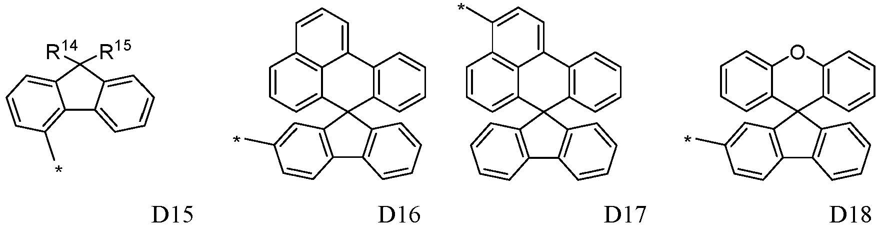 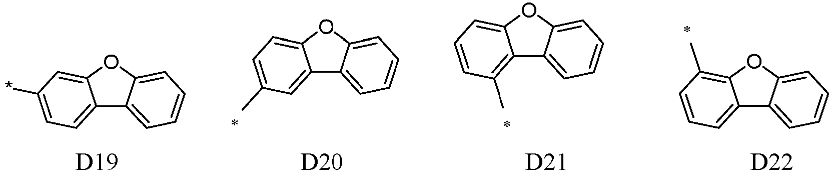 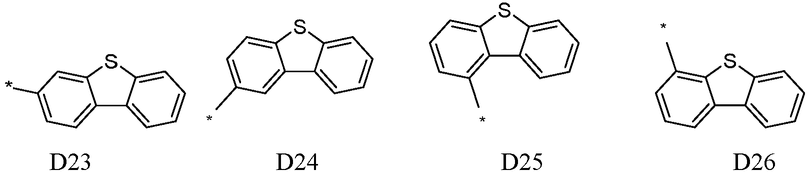 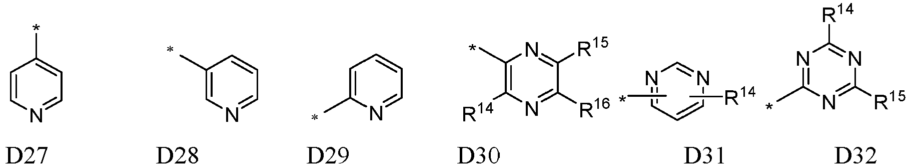 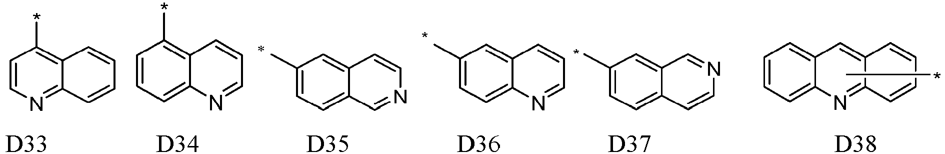 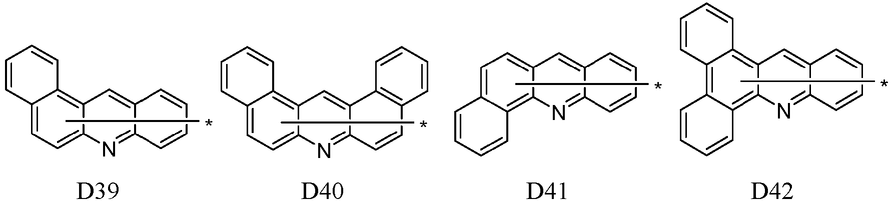 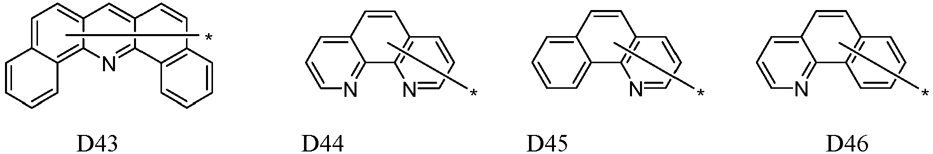 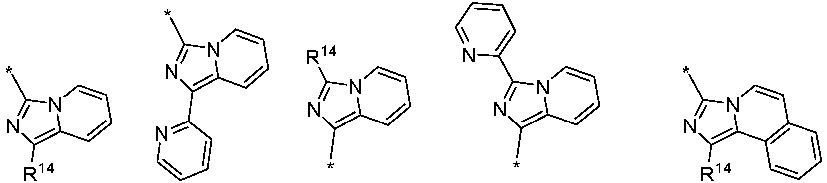 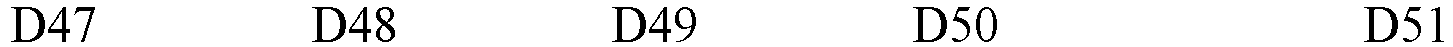 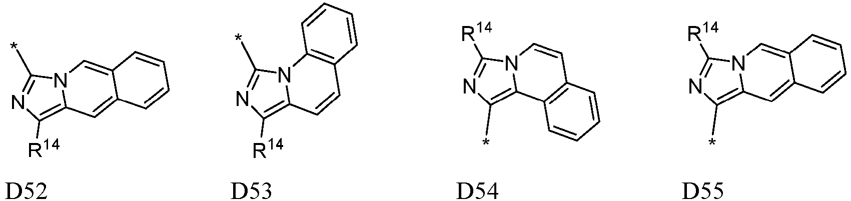 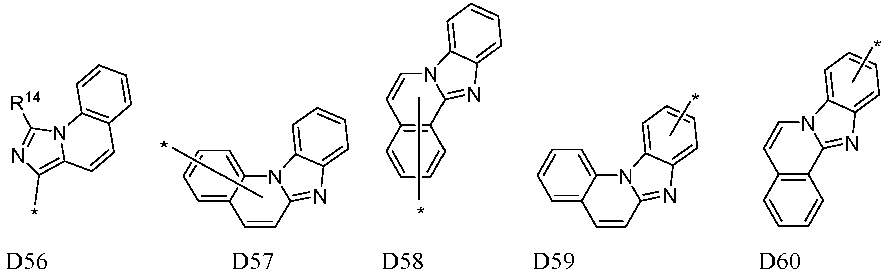 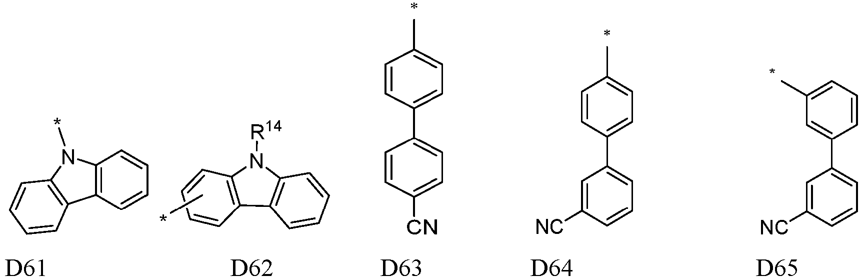 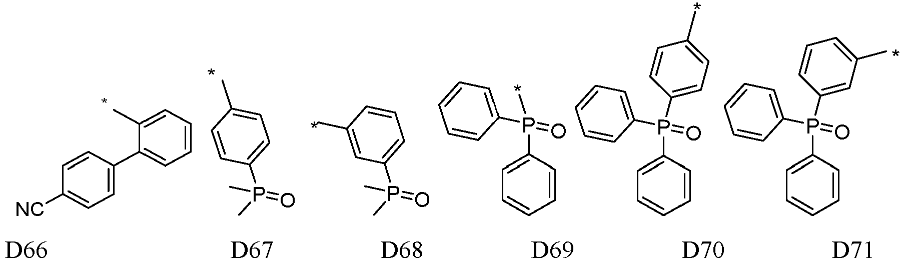 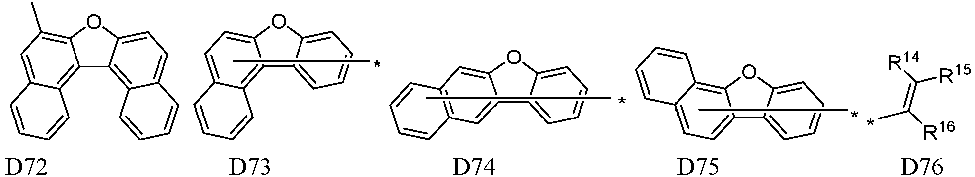 wherein
R¹⁴, R¹⁵ and R¹⁶ are independently selected from H, C₁ to C₁₆ alkyl, C₁ to C₁₆ alkoxy, C₆ to C₁₈ aryl, C₃ to C₂₀ heteroaryl, perfluorinated C₁ to C₁₆ alkyl, perfluorinated C₁ to C₁₆ alkoxy, wherein R¹⁴ and R¹⁵ may be linked via a single bond or a heteroatom to form a ring.

According to one embodiment of the present invention, the moiety Ar is selected from any of the following moieties D1 to D76, and in formula D76 R¹⁴, R¹⁵ and R¹⁶ are independently selected from C₁ to C₁₆ alkyl, C₁ to C₁₆ alkoxy, C₆ to C₁₈ aryl, C₃ to C₂₀ heteroaryl, perfluorinated C₁ to C₁₆ alkyl, perfluorinated C₁ to C₁₆ alkoxy, wherein R¹⁴ and R¹⁵ may be linked via a single bond or a heteroatom to form a ring.

According to one embodiment of the present invention, the moiety Ar is free of styryl, fluorenyl and/or carbazole groups.

According to one embodiment of the present invention, the compound of formula (1) comprises zero or one carbazole group, alternatively compound of formula (1) is free of carbazole groups.

According to one embodiment of the present invention, the compound of formula (1) does not comprise the following structure:

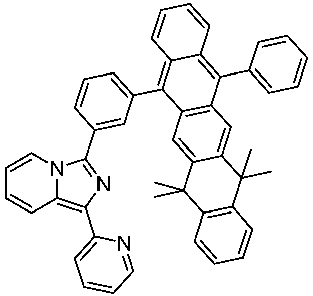

Additionally and/or alternatively according to one embodiment of the present invention, the compound of formula (1) does not comprise the following structures:

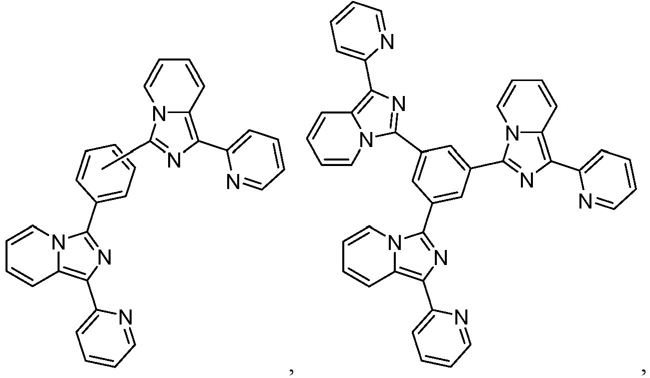

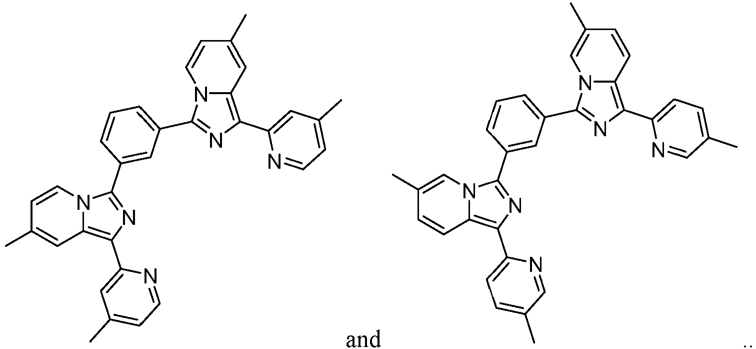

According to one embodiment of the present invention, the compound of formula (1) is selected from the compounds A1 to A37:

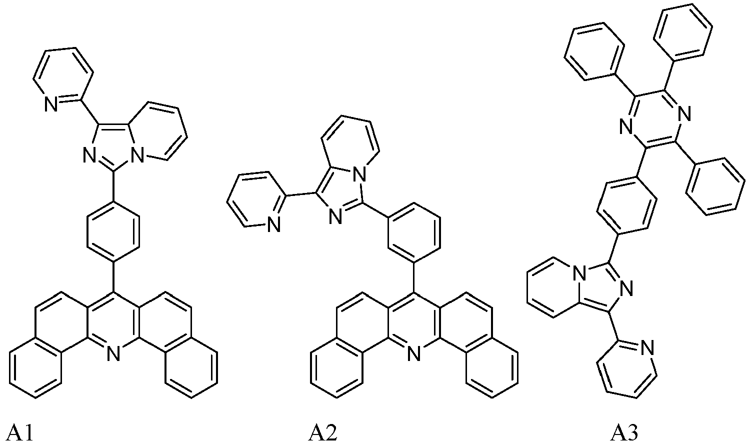

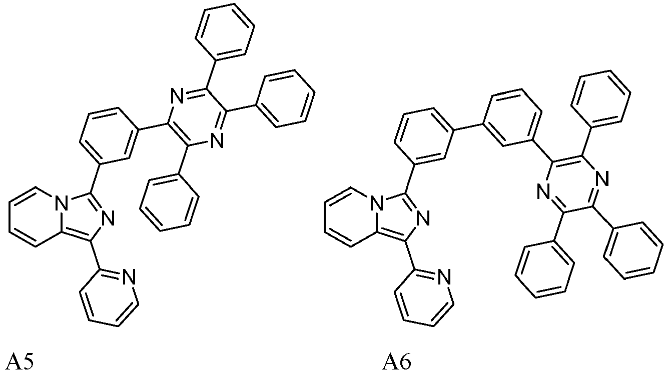

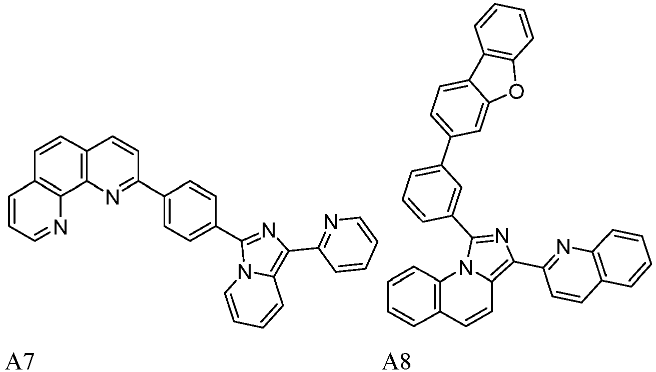

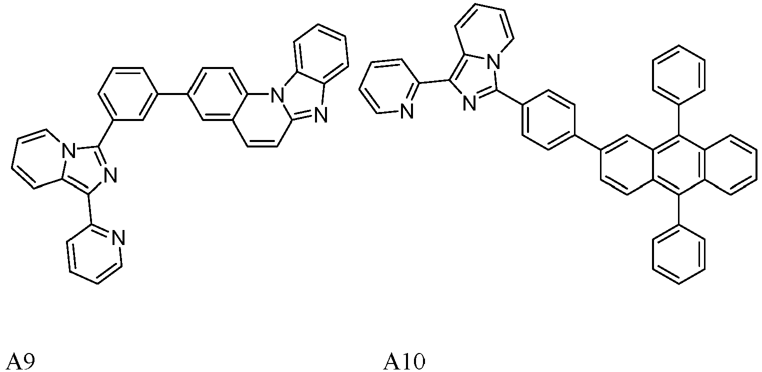

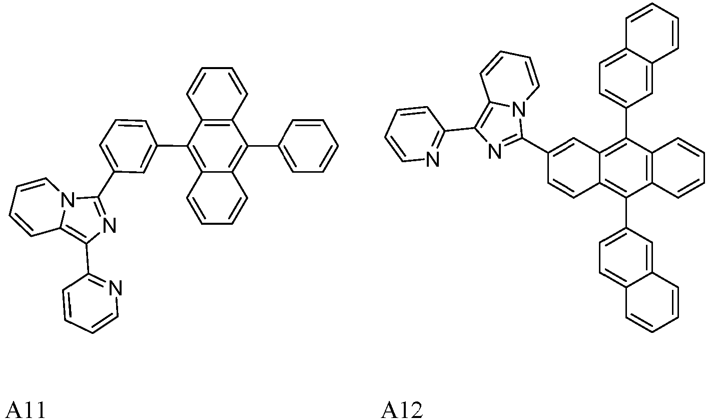

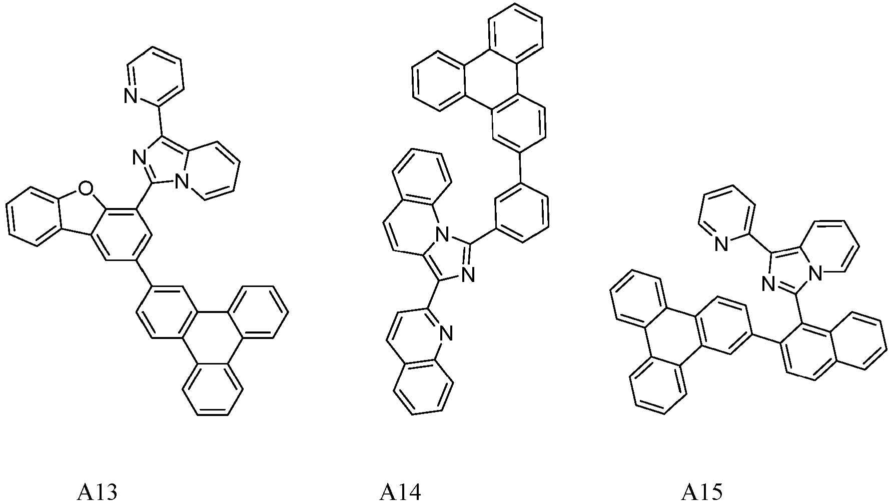

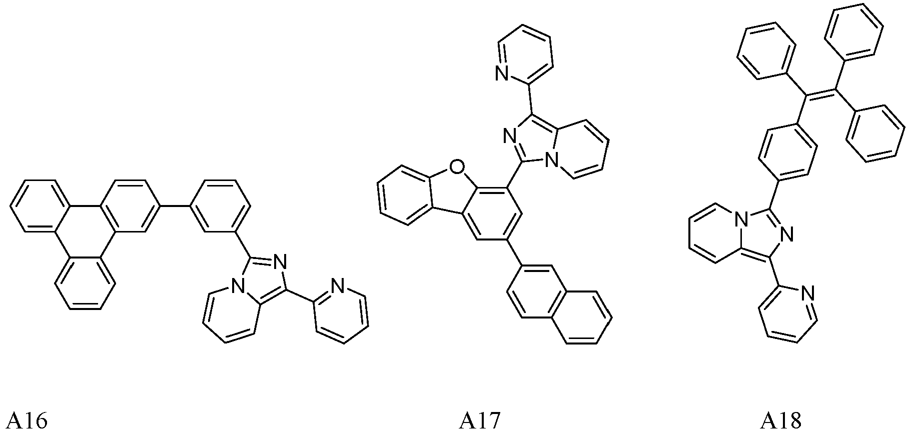

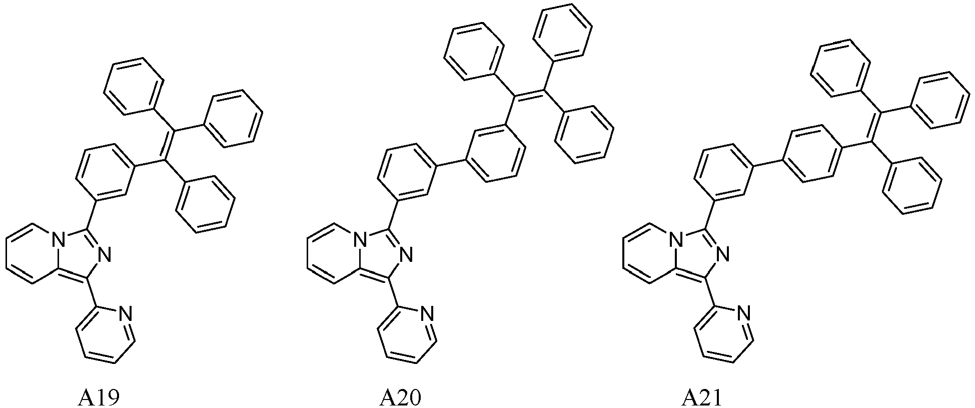

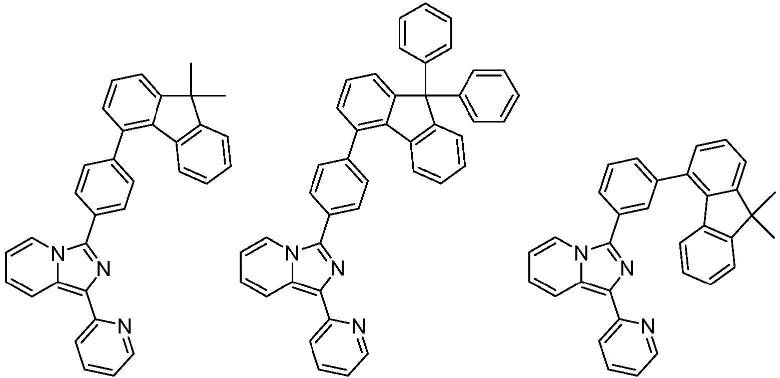

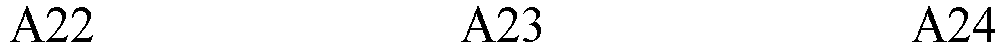

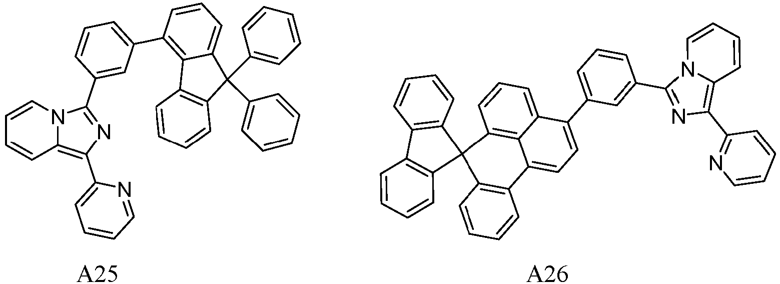

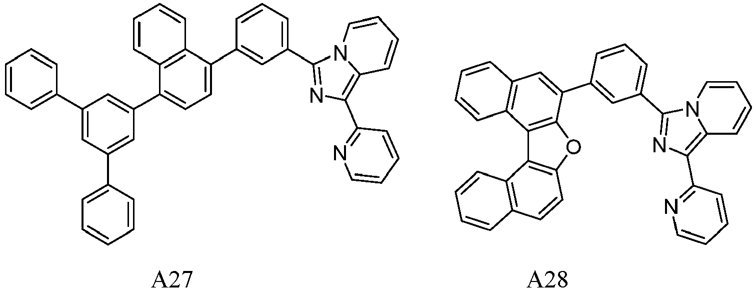

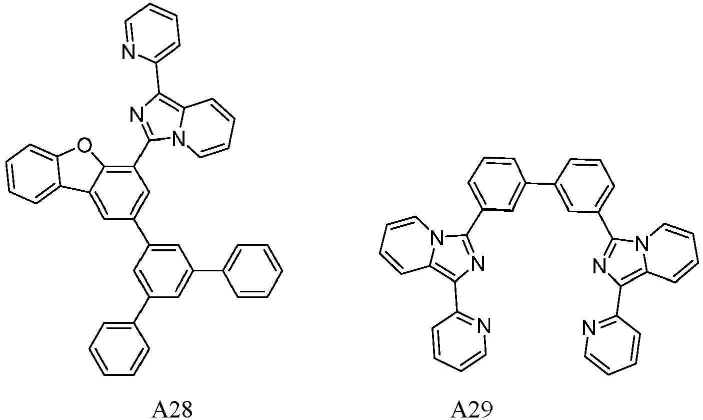

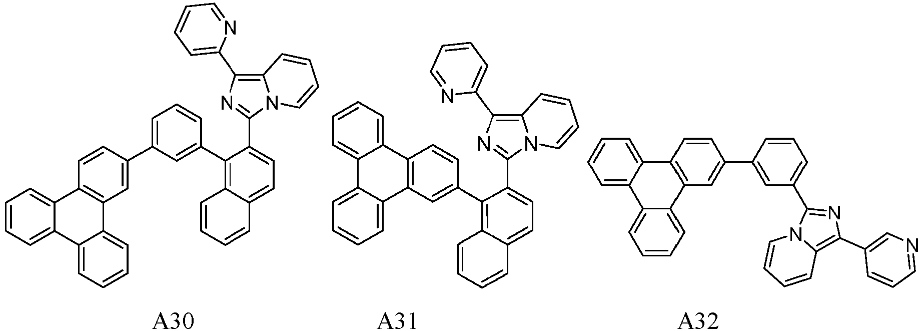

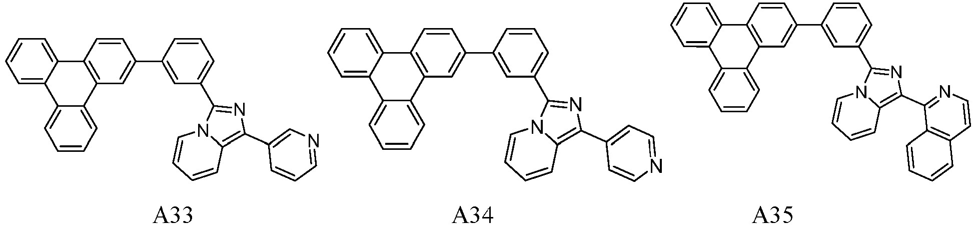

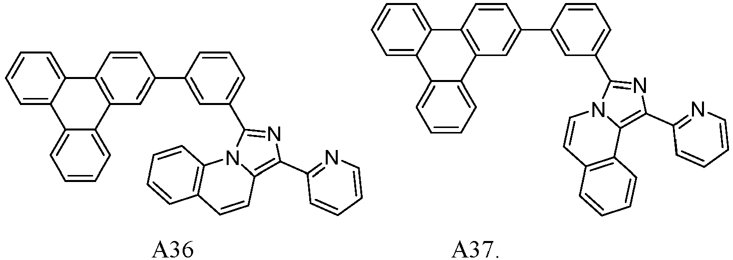

### Redox n-dopant

According to one embodiment of the present invention the organic semiconductor layer of the organic electronic device comprises a redox n-dopant.

Preferably the organic semiconductor layer comprising compound of formula (1) and a redox n-dopant is non-emissive.

Under a redox n-dopant, it is understood a compound which, if embedded into an electron transport matrix, improves, in comparison with the neat matrix under the same physical conditions, the electronic properties of the formed organic material, in particular in terms of electron injection, electron generation and/or electron conductivity. Preferably, the redox n-dopant is non-emissive.

In the context of the present invention "embedded into an electron transport matrix" means the redox n-dopant forms a mixture with the electron transport matrix.

The redox n-dopant may be selected from elemental metals, metal salts, metal complexes and organic radicals.

For the use in consumer electronics, only metals containing stable nuclides or nuclides having very long halftime of radioactive decay might be applicable. As an acceptable level of nuclear stability, the nuclear stability of natural potassium can be taken.

In the context of the present invention, a metal is understood to be a metal in its elemental form, a metal alloy, or in a state of free atoms or metal clusters. It is understood that metals deposited by vacuum thermal evaporation may from a metallic phase, e.g. from a neat bulk metal, vaporize in their elemental form.

It is further understood that if the vaporized elemental metal is deposited together with a covalent matrix, the metal atoms and/or clusters are embedded in the covalent matrix. In other words, it is understood that any metal doped covalent material prepared by vacuum thermal evaporation contains the metal at least partially in its elemental form.

According to one embodiment of the present invention, the organic semiconductor layer of the organic electronic device comprises a metal, preferably selected from alkali metals, alkaline earth metals, rare earth metals and metals of the first transition period Ti, V, Cr and Mn, especially selected from_Li, Na, K, Rb, Cs, Mg, Ca, Sr, Ba, Sm, Eu, Tm, Yb; more preferably from Li, Na, K, Rb, Cs, Mg and Yb, even more preferably from Li, Na, Cs and Yb, most preferably from Li, Na and Yb.

In one embodiment, the redox n-dopant is selected from alkali metal salts and alkali metal complexes; preferably from lithium salts and lithium organic complexes; more preferably from lithium halides and lithium organic chelates; even more preferably from lithium fluoride, a lithium quinolinolate, lithium borate, lithium phenolate, lithium pyridinolate or from a lithium complex with a Schiff base ligand; most preferably,
- the lithium complex has the formula II, III or IV: 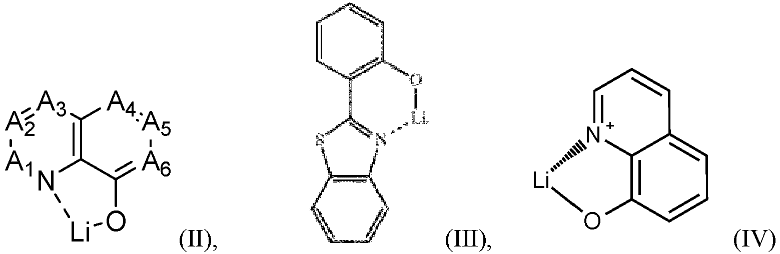 wherein
   A1 to A6 are same or independently selected from CH, CR, N, O;
   R is same or independently selected from hydrogen, halogen, alkyl or aryl or
   heteroaryl with 1 to 20 carbon atoms; and more preferred A1 to A6 are CH,
- the borate based organic ligand is a tetra(1H-pyrazol-1-yl)borate,
- the phenolate is a 2-(pyridin-2-yl)phenolate, a 2-(diphenylphosphoryl)phenolate, an imidazol phenolate, 2-(pyridin-2-yl)phenolate or 2-(1-phenyl-1H-benzo[d]imidazol-2-yl)phenolate,
- the pyridinolate is a 2-(diphenylphosphoryl)pyridin-3-olate,
- the lithium Schiff base has the structure 100, 101, 102 or 103:

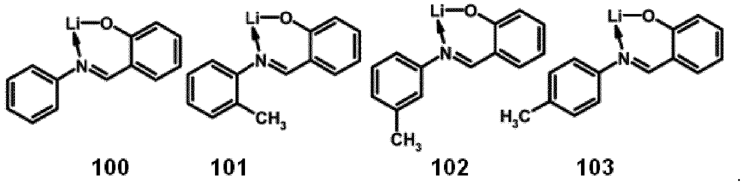

According to one embodiment of the invention, the organic semiconductor layer of the present invention comprises a lithium organic complex, alternatively LiQ.

According to one embodiment of the invention, the organic semiconductor layer of the present invention is an electron transport, electron injection or charge generation layer; alternatively an electron transport or charge generation layer.

According to one embodiment of the invention, the at least one photoactive layer is a light-emitting layer.

According to one embodiment of the invention the organic electronic device comprises a first and a second light-emitting layer, wherein the organic semiconductor layer is arranged between the first and the second light-emitting layer.

According to one embodiment of the invention the organic electronic device comprises a first, a second and a third light-emitting layer, wherein the organic semiconductor layer is arranged between the first and the second light-emitting layer and/or between the second and third light-emitting layer.

According to one embodiment of the invention, the organic semiconductor layer is a charge generation layer, alternatively an n-type charge generation layer.

According to one embodiment of the invention the electronic organic device is an electroluminescent device, preferably an organic light emitting diode.

The present invention furthermore relates to a display device comprising an organic electronic device according to the present invention.

The present invention furthermore relates to a compound of formula (1), according to claim 1, and the following compounds are excluded

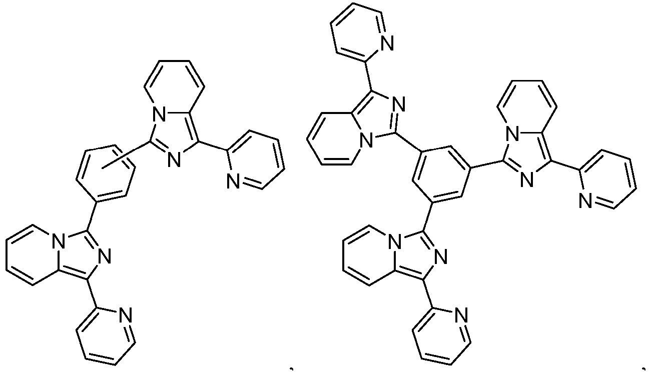

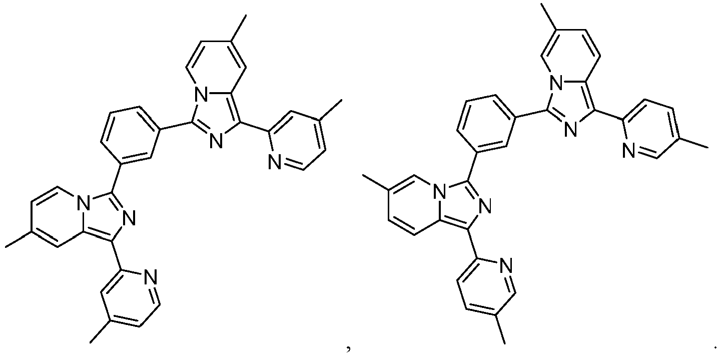

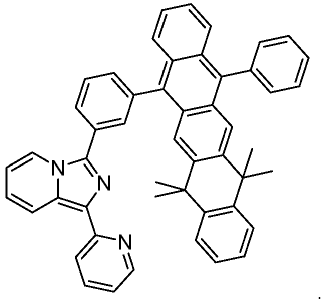

Any specifications of formula (1) as described above in the context of the organic electronic device apply *mutatis mutandis.*

### Further layers

In accordance with the invention, the organic electronic device may comprise, besides the layers already mentioned above, further layers. Exemplary embodiments of respective layers are described in the following:

### Substrate

The substrate may be any substrate that is commonly used in manufacturing of, electronic devices, such as organic light-emitting diodes. If light is to be emitted through the substrate, the substrate shall be a transparent or semitransparent material, for example a glass substrate or a transparent plastic substrate. If light is to be emitted through the top surface, the substrate may be both a transparent as well as a non-transparent material, for example a glass substrate, a plastic substrate, a metal substrate or a silicon substrate.

### Anode electrode

Either a first electrode or a second electrode comprised in the inventive organic electronic device may be an anode electrode. The anode electrode may be formed by depositing or sputtering a material that is used to form the anode electrode. The material used to form the anode electrode may be a high work-function material, so as to facilitate hole injection. The anode material may also be selected from a low work function material (i.e. aluminum). The anode electrode may be a transparent or reflective electrode. Transparent conductive oxides, such as indium tin oxide (ITO), indium zinc oxide (IZO), tin-dioxide (SnO2), aluminum zinc oxide (AlZO) and zinc oxide (ZnO), may be used to form the anode electrode. The anode electrode may also be formed using metals, typically silver (Ag), gold (Au), or metal alloys.

### Hole injection layer

A hole injection layer (HIL) may be formed on the anode electrode by vacuum deposition, spin coating, printing, casting, slot-die coating, Langmuir-Blodgett (LB) deposition, or the like. When the HIL is formed using vacuum deposition, the deposition conditions may vary according to the compound that is used to form the HIL, and the desired structure and thermal properties of the HIL. In general, however, conditions for vacuum deposition may include a deposition temperature of 100° C to 500° C, a pressure of 10⁻⁸ to 10⁻³ Torr (1 Torr equals 133.322 Pa), and a deposition rate of 0.1 to 10 nm/sec.

When the HIL is formed using spin coating or printing, coating conditions may vary according to the compound that is used to form the HIL, and the desired structure and thermal properties of the HIL. For example, the coating conditions may include a coating speed of about 2000 rpm to about 5000 rpm, and a thermal treatment temperature of about 80° C to about 200° C. Thermal treatment removes a solvent after the coating is performed.

The HIL may be formed of any compound that is commonly used to form a HIL. Examples of compounds that may be used to form the HIL include a phthalocyanine compound, such as copper phthalocyanine (CuPc), 4,4',4"-tris (3-methylphenylphenylamino) triphenylamine (m-MTDATA), TDATA, 2T-NATA, polyaniline/dodecylbenzenesulfonic acid (Pani/DBSA), poly(3,4-ethylenedioxythiophene)/poly(4-styrenesulfonate) (PEDOT/PSS), polyaniline/camphor sulfonic acid (Pani/CSA), and polyaniline)/poly(4-styrenesulfonate (PANI/PSS).

The HIL may comprise or consist of p-type dopant and the p-type dopant may be selected from tetrafluoro-tetracyanoquinonedimethane (F4TCNQ), 2,2'-(perfluoronaphthalen-2,6-diylidene) dimalononitrile or 2,2',2"-(cyclopropane-1,2,3-triylidene)tris(2-(p-cyanotetrafluorophenyl)acetonitrile) but not limited hereto. The HIL may be selected from a holetransporting matrix compound doped with a p-type dopant. Typical examples of known doped hole transport materials are: copper phthalocyanine (CuPc), which HOMO level is approximately -5.2 eV, doped with tetrafluoro-tetracyanoquinonedimethane (F4TCNQ), which LUMO level is about -5.2 eV; zinc phthalocyanine (ZnPc) (HOMO = -5.2 eV) doped with F4TCNQ; α-NPD (N,N'-Bis(naphthalen-1-yl)-N,N'-bis(phenyl)-benzidine) doped with F4TCNQ. α-NPD doped with 2,2'-(perfluoronaphthalen-2,6-diylidene) dimalononitrile. The p-type dopant concentrations can be selected from 1 to 20 wt.-%, more preferably from 3 wt.-% to 10 wt.-%.

The thickness of the HIL may be in the range from about 1 nm to about 100 nm, and for example, from about 1 nm to about 25 nm. When the thickness of the HIL is within this range, the HIL may have excellent hole injecting characteristics, without a substantial penalty in driving voltage.

### Hole transport layer

A hole transport layer (HTL) may be formed on the HIL by vacuum deposition, spin coating, slot-die coating, printing, casting, Langmuir-Blodgett (LB) deposition, or the like. When the HTL is formed by vacuum deposition or spin coating, the conditions for deposition and coating may be similar to those for the formation of the HIL. However, the conditions for the vacuum or solution deposition may vary, according to the compound that is used to form the HTL.

The HTL may be formed of any compound that is commonly used to form a HTL. Compounds that can be suitably used are disclosed for example in Yasuhiko Shirota and Hiroshi Kageyama, Chem. Rev. 2007, 107, 953-1010.

Examples of the compound that may be used to form the HTL are: carbazole derivatives, such as N-phenylcarbazole or polyvinylcarbazole; benzidine derivatives, such as N,N'-bis(3-methylphenyl)-N,N'-diphenyl-[1,1-biphenyl]-4,4'-diamine (TPD), or N,N'-di(naphthalen-1-yl)-N,N'-diphenyl benzidine (alpha-NPD); and triphenylamine-based compound, such as 4,4',4"-tris(N-carbazolyl)triphenylamine (TCTA). Among these compounds, TCTA can transport holes and inhibit excitons from being diffused into the EML.

The thickness of the HTL may be in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 160 nm, further about 100 nm to about 160 nm, further about 120 nm to about 140 nm. A preferred thickness of the HTL may be 170 nm to 200 nm.

When the thickness of the HTL is within this range, the HTL may have excellent hole transporting characteristics, without a substantial penalty in driving voltage.

### Electron blocking layer

The function of an electron blocking layer (EBL) is to prevent electrons from being transferred from an emission layer to the hole transport layer and thereby confine electrons to the emission layer. Thereby, efficiency, operating voltage and/or lifetime are improved. Typically, the electron blocking layer comprises a triarylamine compound. The triarylamine compound may have a LUMO level closer to vacuum level than the LUMO level of the hole transport layer. The electron blocking layer may have a HOMO level that is further away from vacuum level compared to the HOMO level of the hole transport layer. The thickness of the electron blocking layer may be selected between 2 and 20 nm.

If the electron blocking layer has a high triplet level, it may also be described as triplet control layer.

The function of the triplet control layer is to reduce quenching of triplets if a phosphorescent green or blue emission layer is used. Thereby, higher efficiency of light emission from a phosphorescent emission layer can be achieved. The triplet control layer is selected from triarylamine compounds with a triplet level above the triplet level of the phosphorescent emitter in the adjacent emission layer. Suitable compounds for the triplet control layer, in particular the triarylamine compounds, are described in EP 2 722 908 A1.

### Photoactive layer (PAL)

The photoactive layer converts an electrical current into photons or photons into an electrical current.

The PAL may be formed on the HTL by vacuum deposition, spin coating, slot-die coating, printing, casting, LB deposition, or the like. When the PAL is formed using vacuum deposition or spin coating, the conditions for deposition and coating may be similar to those for the formation of the HIL. However, the conditions for deposition and coating may vary, according to the compound that is used to form the PAL.

It may be provided that the photoactive layer does not comprise the compound of Formula (1).

The photoactive layer may be a light-emitting layer or a light-absorbing layer.

### Emission layer (EML)

The EML may be formed on the HTL by vacuum deposition, spin coating, slot-die coating, printing, casting, LB deposition, or the like. When the EML is formed using vacuum deposition or spin coating, the conditions for deposition and coating may be similar to those for the formation of the HIL. However, the conditions for deposition and coating may vary, according to the compound that is used to form the EML.

It may be provided that the emission layer does not comprise the compound of Formula (1).

The emission layer (EML) may be formed of a combination of a host and an emitter dopant. Example of the host are Alq3, 4,4'-N,N'-dicarbazole-biphenyl (CBP), poly(n-vinylcarbazole) (PVK), 9,10-di(naphthalene-2-yl)anthracene (ADN), 4,4',4"-tris(carbazol-9-yl)-triphenylamine(TCTA), 1,3,5-tris(N-phenylbenzimidazole-2-yl)benzene (TPBI), 3-tert-butyl-9,10-di-2-naphthylanthracenee (TBADN), distyrylarylene (DSA) and bis(2-(2-hydroxyphenyl)benzo-thiazolate)zinc (Zn(BTZ)2).

The emitter dopant may be a phosphorescent or fluorescent emitter. Phosphorescent emitters and emitters which emit light via a thermally activated delayed fluorescence (TADF) mechanism may be preferred due to their higher efficiency. The emitter may be a small molecule or a polymer.

Examples of red emitter dopants are PtOEP, Ir(piq)3, and Btp2lr(acac), but are not limited thereto. These compounds are phosphorescent emitters, however, fluorescent red emitter dopants could also be used.

Examples of phosphorescent green emitter dopants are Ir(ppy)3 (ppy = phenylpyridine), Ir(ppy)2(acac), Ir(mpyp)3.

Examples of phosphorescent blue emitter dopants are F2Irpic, (F2ppy)2Ir(tmd) and Ir(dfppz)3 and ter-fluorene. 4.4'-bis(4-diphenyl amiostyryl)biphenyl (DPAVBi), 2,5,8,11-tetra-tert-butyl perylene (TBPe) are examples of fluorescent blue emitter dopants.

The amount of the emitter dopant may be in the range from about 0.01 to about 50 parts by weight, based on 100 parts by weight of the host. Alternatively, the emission layer may consist of a light-emitting polymer. The EML may have a thickness of about 10 nm to about 100 nm, for example, from about 20 nm to about 60 nm. When the thickness of the EML is within this range, the EML may have excellent light emission, without a substantial penalty in driving voltage.

### Hole blocking layer (HBL)

A hole blocking layer (HBL) may be formed on the EML, by using vacuum deposition, spin coating, slot-die coating, printing, casting, LB deposition, or the like, in order to prevent the diffusion of holes into the ETL. When the EML comprises a phosphorescent dopant, the HBL may have also a triplet exciton blocking function. The hole blocking layer may be the inventive organic semiconductor layer comprising or consisting of the inventive compound represented by the general Formula (1) as defined above.

The HBL may also be named auxiliary ETL or a-ETL.

When the HBL is formed using vacuum deposition or spin coating, the conditions for deposition and coating may be similar to those for the formation of the HIL. However, the conditions for deposition and coating may vary, according to the compound that is used to form the HBL. Any compound that is commonly used to form a HBL may be used. Examples of compounds for forming the HBL include oxadiazole derivatives, triazole derivatives, and phenanthroline derivatives.

The HBL may have a thickness in the range from about 5 nm to about 100 nm, for example, from about 10 nm to about 30 nm. When the thickness of the HBL is within this range, the HBL may have excellent hole-blocking properties, without a substantial penalty in driving voltage.

The hole blocking layer may also be described as a-ETL or auxiliary ETL.

According to an embodiment, a hole blocking layer is arranged between the at least one photoactive layer and the organic semiconductor layer comprising compound of formula (1).

According to an embodiment, a hole blocking layer is arranged between the at least one photoactive layer and the organic semiconductor layer comprising compound of formula (1), wherein the organic semiconductor layer comprising compound of formula (1) further comprises a redox n-dopant.

According to an embodiment, a hole blocking layer is arranged between the at least one photoactive layer and the organic semiconductor layer comprising compound of formula (1), wherein the organic semiconductor layer comprising compound of formula (1) further comprises a metal or a metal organic complex, alternatively a metal or a lithium organic complex.

According to an embodiment, a hole blocking layer is arranged between the at least one photoactive layer and the organic semiconductor layer comprising compound of formula (1), wherein the organic semiconductor layer comprising compound of formula (1) further comprises a metal.

### Electron transport layer (ETL)

The OLED according to the present invention may comprise an electron transport layer (ETL). In accordance with one preferred embodiment of the invention, the electron transport layer may be the inventive organic semiconductor layer comprising the inventive compound represented by the general Formula (1) as defined herein.

According to various embodiments the OLED may comprise an electron transport layer or an electron transport layer stack comprising at least a first electron transport layer and at least a second electron transport layer.

By suitably adjusting energy levels of particular layers of the ETL, the injection and transport of the electrons may be controlled, and the holes may be efficiently blocked. Thus, the OLED may have long lifetime.

The electron transport layer of the organic electronic device may comprise the compound represented by general Formula (1) as defined above as the organic electron transport matrix (ETM) material. The electron transport layer may comprise, besides or instead of the compound represented by the general Formula (1), further ETM materials known in the art. Likewise, the electron transport layer may comprise as the only electron transport matrix material the compound represented by general Formula (1). In case that the inventive organic electronic device comprises more than one electron transport layers, the compound represented by the general Formula (1) may be comprised in only one of the electron transport layers, in more than one of the electron transport layers or in all of the electron transport layers. In accordance with the invention, the electron transport layer may comprise, besides the ETM material, at least one additive as defined below.

Further, the electron transport layer may comprise one or more n-type dopants. The additive may be an n-type dopant. The additive can be alkali metal, alkali metal compound, alkaline earth metal, alkaline earth metal compound, transition metal, transition metal compound or a rare earth metal. In another embodiment, the metal can be one selected from a group consisting of Li, Na, K, Rb, Cs, Mg, Ca, Sr, Ba, La, Ce, Sm, Eu, Tb, Dy, and Yb. In another emdodiment, the n-type dopant can be one selected from a group consisting of Cs, K, Rb, Mg, Na, Ca, Sr, Eu and Yb. In an embodiment the alkali metal compound may be 8-Hydroxyquinolinolato-lithium (LiQ), Lithium tetra(1H-pyrazol-1-yl)borate or Lithium 2-(diphenylphosphoryl)phenolate. Suitable compounds for the ETM (which may be used in addition to the inventive compound represented by the general Formula (1) as defined above) are not particularly limited. In one embodiment, the electron transport matrix compounds consist of covalently bound atoms. Preferably, the electron transport matrix compound comprises a conjugated system of at least 6, more preferably of at least 10 delocalized electrons. In one embodiment, the conjugated system of delocalized electrons may be comprised in aromatic or heteroaromatic structural moieties, as disclosed e.g. in documents EP 1 970 371 A1 or WO 2013/079217 A1.

### Electron injection layer (EIL)

An optional EIL, which may facilitates injection of electrons from the cathode, may be formed on the ETL, preferably directly on the electron transport layer. Examples of materials for forming the EIL include lithium 8-hydroxyquinolinolate (LiQ), LiF, NaCl, CsF, Li2O, BaO, Ca, Ba, Yb, Mg which are known in the art. Deposition and coating conditions for forming the EIL are similar to those for formation of the HIL, although the deposition and coating conditions may vary, according to the material that is used to form the EIL. The EIL may be the organic semiconductor layer comprising the compound of Formula (1).

The thickness of the EIL may be in the range from about 0.1 nm to about 10 nm, for example, in the range from about 0.5 nm to about 9 nm. When the thickness of the EIL is within this range, the EIL may have satisfactory electron-injecting properties, without a substantial penalty in driving voltage.

### Cathode electrode

The cathode electrode is formed on the EIL if present. The cathode electrode may be formed of a metal, an alloy, an electrically conductive compound, or a mixture thereof. The cathode electrode may have a low work function. For example, the cathode electrode may be formed of lithium (Li), magnesium (Mg), aluminum (Al), aluminum (Al)-lithium (Li), calcium (Ca), barium (Ba), ytterbium (Yb), magnesium (Mg)-indium (In), magnesium (Mg)-silver (Ag), or the like. Alternatively, the cathode electrode may be formed of a transparent conductive oxide, such as ITO or IZO.

The thickness of the cathode electrode may be in the range from about 5 nm to about 1000 nm, for example, in the range from about 10 nm to about 100 nm. When the thickness of the cathode electrode is in the range from about 5 nm to about 50 nm, the cathode electrode may be transparent or semitransparent even if formed from a metal or metal alloy.

It is to be understood that the cathode electrode is not part of an electron injection layer or the electron transport layer.

### Charge generation layer/hole generating layer

The charge generation layer (CGL) may comprise a p- type and an n-type layer. An interlayer may be arranged between the p-type layer and the n-type layer.

Typically, the charge generation layer is a pn junction joining an n-type charge generation layer (electron generating layer) and a hole generating layer. The n-side of the pn junction generates electrons and injects them into the layer which is adjacent in the direction to the anode. Analogously, the p-side of the p-n junction generates holes and injects them into the layer which is adjacent in the direction to the cathode.

Charge generating layers are used in tandem and stacked devices, for example, in tandem or stacked OLEDs comprising, between two electrodes, two or more emission layers. In a tandem or stacked OLED comprising two emission layers, the n-type charge generation layer provides electrons for the first light emission layer arranged near the anode, while the hole generating layer provides holes to the second light emission layer arranged between the first emission layer and the cathode.

Suitable matrix materials for the hole generating layer may be materials conventionally used as hole injection and/or hole transport matrix materials. Also, p-type dopant used for the hole generating layer can employ conventional materials. For example, the p-type dopant can be one selected from a group consisting of tetrafluore-7,7,8,8-tetracyanoquinodimethane (F4-TCNQ), derivatives of tetracyanoquinodimethane, radialene derivatives, iodine, FeCl3, FeF3, and SbCl5. Also, the host can be one selected from a group consisting of N,N'-di(naphthalen-1-yl)-N,N-diphenyl-benzidine (NPB), N,N'-diphenyl-N,N'-bis(3-methylphenyl)-1,1-biphenyl-4,4'-diamine (TPD) and N,N',N'-tetranaphthyl-benzidine (TNB). The p-type charge generation layer may consist of CNHAT.

The n-type charge generating layer may be the layer comprising the compound of Formula (1). The n-type charge generation layer can be layer of a neat n-type dopant, for example of a metal, or can consist of an organic matrix material doped with the n-type dopant. In one embodiment, the n-type dopant can be alkali metal, alkali metal compound, alkaline earth metal, alkaline earth metal compound, a transition metal, a transition metal compound or a rare earth metal. In another embodiment, the metal can be one selected from a group consisting of Li, Na, K, Rb, Cs, Mg, Ca, Sr, Ba, La, Ce, Sm, Eu, Tb, Dy, and Yb. More specifically, the n-type dopant can be one selected from a group consisting of Li, Cs, K, Rb, Mg, Na, Ca, Sr, Eu and Yb. Suitable matrix materials for the electron generating layer may be the materials conventionally used as matrix materials for electron injection or electron transport layers. The matrix material can be for example one selected from a group consisting of triazine compounds, hydroxyquinoline derivatives like tris(8-hydroxyquinoline)aluminum, benzazole derivatives, and silole derivatives.

The hole generating layer is arranged in direct contact to the n-type charge generation layer.

According to one aspect of the present invention, the organic semiconductor layer is arranged between the first and second emission layer and further comprises a redox n-dopant.

According to one aspect of the present invention, the organic semiconductor layer is arranged between the first and second emission layer and further comprises a metal.

According to one aspect of the present invention, the organic semiconductor layer is arranged between the first and second emission layer and further comprises a metal selected from alkali, alkaline earth and rare earth metals.

According to one aspect of the present invention, the organic semiconductor layer comprising compound of formula (1) is arranged between the first and second emission layer and a further organic semiconductor layer comprising compound of formula (1) is arranged between the second emission layer and the cathode.

According to one aspect of the present invention, the organic semiconductor layer comprising compound of formula (1) is arranged between the first and second emission layer and a further organic semiconductor layer comprising compound of formula (1) is arranged between the second emission layer and the cathode; wherein the organic semiconductor layer comprising compound of formula (1) further comprises a redox n-dopant.

According to one aspect of the present invention, the organic semiconductor layer comprising compound of formula (1) is arranged between the first and second emission layer and a further organic semiconductor layer comprising compound of formula (1) is arranged between the second emission layer and the cathode; wherein the further organic semiconductor layer comprising compound of formula (1) further comprises a redox n-dopant.

According to one aspect of the present invention, the organic semiconductor layer comprising compound of formula (1) is arranged between the first and second emission layer and a further organic semiconductor layer comprising compound of formula (1) is arranged between the second emission layer and the cathode; wherein the organic semiconductor layer comprising compound of formula (1) further comprises a redox n-dopant, and the further organic semiconductor layer comprising compound of formula (1) further comprises a redox n-dopant.

According to one aspect of the present invention, the organic semiconductor layer comprising compound of formula (1) is arranged between the first and second emission layer and a further organic semiconductor layer comprising compound of formula (1) is arranged between the second emission layer and the cathode; wherein the organic semiconductor layer comprising compound of formula (1) further comprises a metal, and the further organic semiconductor layer comprising compound of formula (1) further comprises a redox n-dopant.

### Organic light-emitting diode (OLED)

The organic electronic device according to the invention may be an organic light-emitting device.

According to one aspect of the present invention, there is provided an organic light-emitting diode (OLED) comprising: a substrate; an anode electrode formed on the substrate; a hole injection layer, a hole transport layer, an emission layer, an organic semiconductor layer comprising a compound of Formula (1) and a cathode electrode.

According to another aspect of the present invention, there is provided an OLED comprising: a substrate; an anode electrode formed on the substrate; a hole injection layer, a hole transport layer, an electron blocking layer, an emission layer, a hole blocking layer, an organic semiconductor layer comprising a compound of Formula (1) and a cathode electrode.

According to another aspect of the present invention, there is provided an OLED comprising: a substrate; an anode electrode formed on the substrate; a hole injection layer, a hole transport layer, an electron blocking layer, an emission layer, a hole blocking layer, an organic semiconductor layer comprising a compound of Formula (1), an electron injection layer, and a cathode electrode.

According to various embodiments of the present invention, there may be provided OLEDs layers arranged between the above mentioned layers, on the substrate or on the top electrode.

According to one aspect, the OLED can comprise a layer structure of a substrate that is adjacent arranged to an anode electrode, the anode electrode is adjacent arranged to a first hole injection layer, the first hole injection layer is adjacent arranged to a first hole transport layer, the first hole transport layer is adjacent arranged to a first electron blocking layer, the first electron blocking layer is adjacent arranged to a first emission layer, the first emission layer is adjacent arranged to a first electron transport layer, the first electron transport layer is adjacent arranged to an n-type charge generation layer, the n-type charge generation layer is adjacent arranged to a hole generating layer, the hole generating layer is adjacent arranged to a second hole transport layer, the second hole transport layer is adjacent arranged to a second electron blocking layer, the second electron blocking layer is adjacent arranged to a second emission layer, between the second emission layer and the cathode electrode an optional electron transport layer and/or an optional injection layer are arranged.

The organic semiconductor layer according to the invention may be the electron transport layer, first electron transport layer, n-type charge generation layer and/or second electron transport layer.

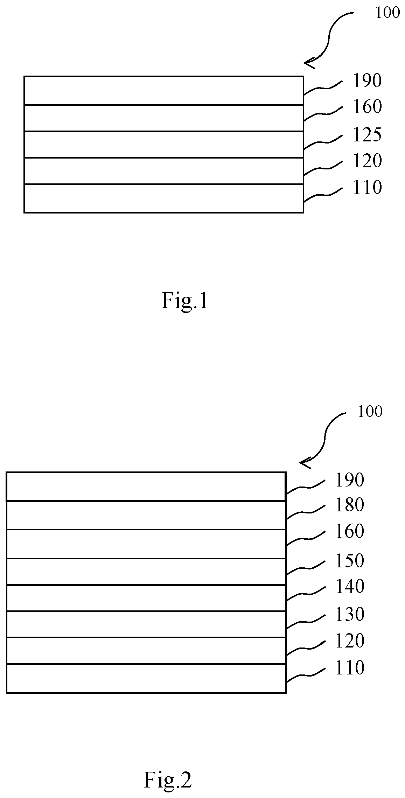

For example, the OLED according to Fig. 2 may be formed by a process, wherein on a substrate (110), an anode (120), a hole injection layer (130), a hole transport layer (140), an electron blocking layer (145), an emission layer (150), a hole blocking layer (155), an electron transport layer (160), an electron injection layer (180) and the cathode electrode (190) are subsequently formed in that order.

### Organic electronic device

The organic electronic device according to the invention may be a light emitting device, or a photovoltaic cell, and preferably a light emitting device.

According to another aspect of the present invention, there is provided a method of manufacturing an organic electronic device, the method using:
- at least one deposition source, preferably two deposition sources and more preferred at least three deposition sources.

The methods for deposition that can be suitable comprise:
- deposition via vacuum thermal evaporation;
- deposition via solution processing, preferably the processing is selected from spincoating, printing, casting; and/or
- slot-die coating.

According to various embodiments of the present invention, there is provided a method using:
- a first deposition source to release the compound of Formula (1) according to the invention, and
- a second deposition source to release the metal, a metal salt or an alkali or alkaline earth metal complex; alternatively an organic alkali or alkaline earth metal complex; alternatively 8-hydroxyquinolinolato lithium;
the method comprising the steps of forming the organic semiconductor layer; whereby for an organic light-emitting diode (OLED):
- the organic semiconductor layer is formed by releasing the compound of Formula (1) according to the invention from the first deposition source and a metal, a metal salt or an alkali or alkaline earth metal complex; alternatively an organic alkali or alkaline earth metal complex; alternatively 8-hydroxyquinolinolato lithium, from the second deposition source.

According to various embodiments of the present invention, the method may further include forming on the anode electrode, an emission layer and at least one layer selected from the group consisting of forming a hole injection layer, forming a hole transport layer, or forming a hole blocking layer, between the anode electrode and the first electron transport layer.

According to various embodiments of the present invention, the method may further include the steps for forming an organic light-emitting diode (OLED), wherein
- on a substrate a first anode electrode is formed,
- on the first anode electrode an emission layer is formed,
- on the emission layer an electron transport layer stack is formed, optionally a hole blocking layer is formed on the emission layer and an organic semiconductor layer is formed,
- and finally a cathode electrode is formed,
- optional a hole injection layer, a hole transport layer, and a hole blocking layer, formed in that order between the first anode electrode and the emission layer,
- optional an electron injection layer is formed between the organic semiconductor layer and the cathode electrode.

According to various embodiments of the present invention, the method may further comprise forming an electron injection layer on the organic semiconductor layer. However, according to various embodiments of the OLED of the present invention, the OLED may not comprise an electron injection layer.

According to various embodiments, the OLED may have the following layer structure, wherein the layers having the following order:
anode, hole injection layer, first hole transport layer, second hole transport layer, emission layer, optional hole blocking layer, organic semiconductor layer comprising a compound of Formula (1) according to the invention, optional electron injection layer, and cathode.

According to another aspect of the invention, it is provided an electronic device comprising at least one organic light emitting device according to any embodiment described throughout this application, preferably, the electronic device comprises the organic light emitting diode in one of embodiments described throughout this application. More preferably, the electronic device is a display device.

In one embodiment, the organic electronic device according to the invention comprising an organic semiconductor layer comprising a compound according to Formula (1) may further comprise a layer comprising a radialene compound and/or a quinodimethane compound.

In one embodiment, the radialene compound and/or the quinodimethane compound may be substituted with one or more halogen atoms and/or with one or more electron withdrawing groups. Electron withdrawing groups can be selected from nitrile groups, halogenated alkyl groups, alternatively from perhalogenated alkyl groups, alternatively from perfluorinated alkyl groups. Other examples of electron withdrawing groups may be acyl, sulfonyl groups or phosphoryl groups.

Alternatively, acyl groups, sulfonyl groups and/or phosphoryl groups may comprise halogenated and/or perhalogenated hydrocarbyl. In one embodiment, the perhalogenated hydrocarbyl may be a perfluorinated hydrocarbyl. Examples of a perfluorinated hydrocarbyl can be perfluormethyl, perfluorethyl, perfluorpropyl, perfluorisopropyl, perfluorobutyl, perfluorophenyl, perfluorotolyl; examples of sulfonyl groups comprising a halogenated hydrocarbyl may be trifluoromethylsulfonyl, pentafluoroethylsulfonyl, pentafluorophenylsulfonyl, heptafluoropropylsufonyl, nonafluorobutylsulfonyl, and like.

In one embodiment, the radialene and/or the quinodimethane compound may be comprised in a hole injection, hole transporting and/or a hole generation layer.

In one embodiment, the radialene compound may have Formula (XX) and/or the quinodimethane compound may have Formula (XXIa) or (XXIb): 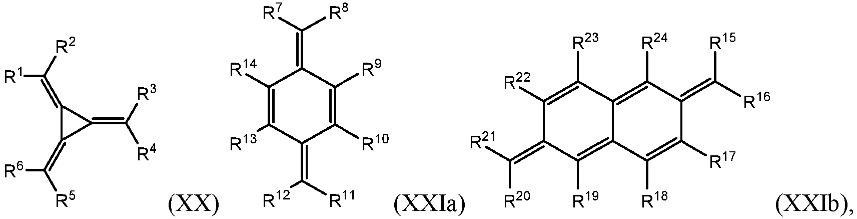 wherein (as an exception different to the description above) R¹, R², R³, R⁴, R⁵, R⁶, R⁷, R⁸, R¹¹, R¹², R¹⁵, R¹⁶, R²⁰, R²¹ are independently selected from above mentioned electron withdrawing groups and R⁹, R¹⁹, R¹³, R¹⁴, R¹⁷, R¹⁸, R¹⁹, R²², R²³ and R²⁴ are independently selected from H, halogen and above mentioned electron withdrawing groups.

According to one embodiment of the present invention, the organic semiconductor layer comprising compound of formula (1) is adjacent to a layer comprising a compound of formula (XX), (XXIa) or (XXIb).

According to one embodiment of the present invention, the organic semiconductor layer comprising compound of formula (1) is in direct contact to a layer comprising a compound of formula (XX), (XXIa) or (XXIb).

Hereinafter, the embodiments are illustrated in more detail with reference to examples. However, the present disclosure is not limited to the following examples. Reference will now be made in detail to the exemplary aspects.

### Description of the Drawings

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

Additional details, characteristics and advantages of the object of the invention are disclosed in the dependent claims and the following description of the respective figures which in an exemplary fashion show preferred embodiments according to the invention. Any embodiment does not necessarily represent the full scope of the invention, however, and reference is made therefore to the claims and herein for interpreting the scope of the invention. It is to be understood that both the foregoing general description and the following detailed description are exemplary and explanatory only and are intended to provide further explanation of the present invention as claimed.
FIG. 2 is a schematic sectional view of an organic electronic device, according to an exemplary embodiment of the present invention;
FIG. 2 is a schematic sectional view of an organic light-emitting diode (OLED), according to an exemplary embodiment of the present invention;
FIG. 3 is a schematic sectional view of an OLED, according to an exemplary embodiment of the present invention.
FIG. 4 is a schematic sectional view of an OLED comprising a charge generation layer and two emission layers, according to an exemplary embodiment of the present invention.

Hereinafter, the figures are illustrated in more detail with reference to examples. However, the present disclosure is not limited to the following figures.

Herein, when a first element is referred to as being formed or disposed "on" or "onto" a second element, the first element can be disposed directly on the second element, or one or more other elements may be disposed there between. When a first element is referred to as being formed or disposed "directly on" or "directly onto" a second element, no other elements are disposed there between.

FIG. 1 is a schematic sectional view of an organic electronic device 100, according to an exemplary embodiment of the present invention. The organic electronic device 100 includes a substrate 110, an anode 120, a photoactive layer (PAL) 125, an organic semiconductor layer comprising a compound of formula (1) 160. The organic semiconductor layer comprising compound of formula (1) 160 is formed on the PAL 125. Onto the organic semiconductor layer 160, a cathode 190 is disposed.

FIG. 2 is a schematic sectional view of an organic light-emitting diode (OLED) 100, according to an exemplary embodiment of the present invention. The OLED 100 includes a substrate 110, an anode 120, a hole injection layer (HIL) 130, a hole transport layer (HTL) 140, an emission layer (EML) 150, an electron transport layer (ETL) 160. The electron transport layer (ETL) 160 is formed on the EML 150. Onto the electron transport layer (ETL) 160, an electron injection layer (EIL) 180 is disposed. The cathode 190 is disposed directly onto the electron injection layer (EIL) 180.

Instead of a single electron transport layer 160, optionally an electron transport layer stack (ETL) can be used.

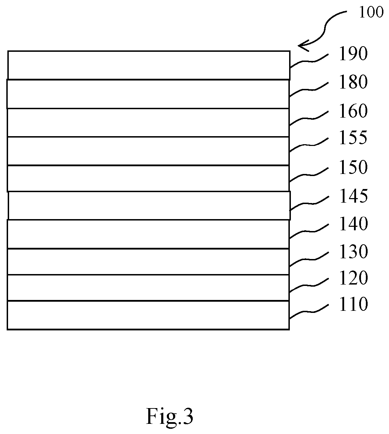

Fig. 3 is a schematic sectional view of an OLED 100, according to another exemplary embodiment of the present invention. Fig. 2 differs from Fig. 1 in that the OLED 100 of Fig. 2 comprises an electron blocking layer (EBL) 145 and a hole blocking layer (HBL) 155.

Referring to Fig. 3, the OLED 100 includes a substrate 110, an anode 120, a hole injection layer (HIL) 130, a hole transport layer (HTL) 140, an electron blocking layer (EBL) 145, an emission layer (EML) 150, a hole blocking layer (HBL) 155, an electron transport layer (ETL) 160, an electron injection layer (EIL) 180 and a cathode electrode 190.

Preferably, the organic semiconductor layer comprising a compound of Formula (1) may be an ETL.

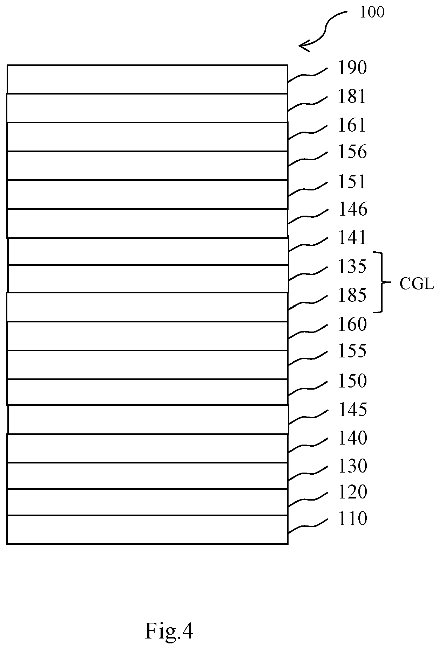

Fig. 4 is a schematic sectional view of an OLED 100, according to another exemplary embodiment of the present invention. Fig. 4 differs from Fig. 3 in that the OLED 100 of Fig. 4 further comprises a charge generation layer (CGL) and a second emission layer (151).

Referring to Fig. 4, the OLED 100 includes a substrate 110, an anode 120, a first hole injection layer (HIL) 130, a first hole transport layer (HTL) 140, a first electron blocking layer (EBL) 145, a first emission layer (EML) 150, a first hole blocking layer (HBL) 155, a first electron transport layer (ETL) 160, an n-type charge generation layer (n-type CGL) 185, a hole generating layer (p-type charge generation layer; p-type GCL) 135, a second hole transport layer (HTL) 141, a second electron blocking layer (EBL) 146, a second emission layer (EML) 151, a second hole blocking layer (EBL) 156, a second electron transport layer (ETL) 161, a second electron injection layer (EIL) 181 and a cathode 190.

Preferably, the organic semiconductor layer comprising a compound of Formula (1) may be an n-type CGL.

Preferably, the organic semiconductor layer comprising a compound of Formula (1) may be the first ETL, n-type CGL and/or second ETL.

While not shown in Fig. 1, Fig. 2, Fig. 3 and Fig. 4, a sealing layer may further be formed on the cathode electrodes 190, in order to seal the OLEDs 100. In addition, various other modifications may be applied thereto.

Hereinafter, the embodiments are illustrated in more detail with reference to examples. However, the present disclosure is not limited to the following examples.

### Detailed description

The invention is furthermore illustrated by the following examples which are illustrative only and non-binding.

In the following the preparation of several inventive compounds is shown, using the following General Method:

### General method:

A flask was flushed with nitrogen and charged with both starting materials (cf. Table 1) in a 1:1 ratio. The organic solvent was added and the mixture was de-aerated. A second flask was charged with the base and water and was de-aerated as well. The aqueous base was added to the starting materials under nitrogen and the reaction was started by the addition of the catalyst. The reaction mixture was heated to the given temperature until TLC showed full conversion. The mixture was then cooled to room temperature and the product was purified according to the methods given in Table 2:

**Table 1: Starting materials for the reactions according to General Method:**

| No | Starting material 1 | Starting material 2 |
|---|---|---|
| A2 | 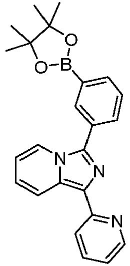 | 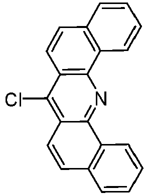 |
| A3 | 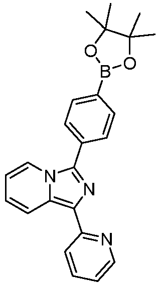 | 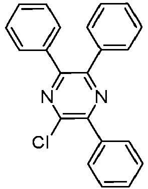 |
| A7 | 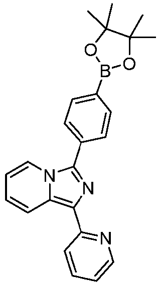 | 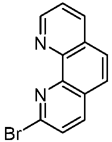 |
| A8 | 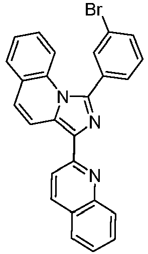 | 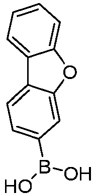 |
| A10 | 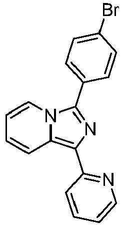 | 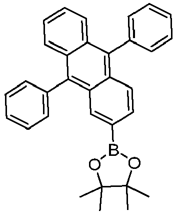 |
| A11 | 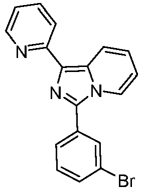 | 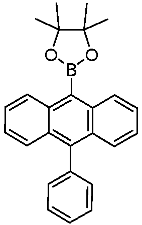 |
| A13 | 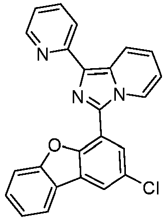 | 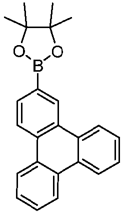 |
| A14 | 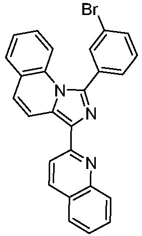 | 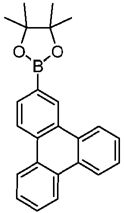 |
| A16 | 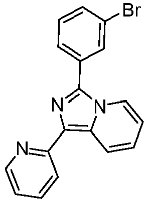 | 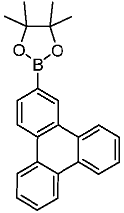 |

**Table 2: Reaction conditions for the reactions of General Method A**

| No | Catalyst / Base | Solvent Temp. | Work-up and purification | Amount (Yield) ESI m/z |
|---|---|---|---|---|
| A2 | Tetrakis (triphenylphosphine) palladium(0) 2mol%, Potassium carbonate 2eq, 2M | 1,4-dioxane/ H2O 8/1 80 °C | product precipitates, washed with water and n-hexane , dissolved in CHCl3, filtered through SiO2 and precipitated with hexane, recrystallized from chlorobenzene | 8.1g (52%) |
| | | | | m/z = 549 [M+H]⁺ |
| A3 | Tetrakis (triphenyl phosphine) palladium(0) 2mol%, Potassium carbonate 2eq, 2M | 1,4-dioxane/ H2O 4/1 80 °C | product precipitates, washed with 1,4-dioxane, water and MeOH, dissolved in DCM and filtered through Florisil^{®}, recrystallized from chlorobenzene | 19.7g (97%) |
| | | | | m/z = 578 [M+H]⁺ |
| A7 | Dichloro [1,1'-bis(diphenylphosphi no)ferrocene] palladium(II) 4,5mol%, Potassium carbonate 2eq, 2M | 1,4-dioxane/ H2O 6/1 80°C | product precipitates, washed with water and n-hexane, dissolved in hot chlorobenzene, filtered through Alox N and allowed to crystallize | 4.5 g (26%) |
| | | | | m/z = 472 [M+Na]⁺ |
| A8 | Dichloro[1,1'-bis(diphenylphosphi no)ferrocene]palladi um(II) 3mol%, Potassium carbonate 2eq, 2M | toluene/THF/ H2O 5/2/1 80 °C | product precipitates, washed with water and n-hexane, soxhlet with THF, precipitated by addition of n-hexane | 4.85g (38%) |
| | | | | m/z = 538 [M+H]⁺ |
| A10 | Dichloro[1,1'-bis(diphenylphosphino)ferrocene]palladi um(II) 3mol%, Potassium carbonate 2eq, 2M | 1,4-dioxane/ H2O 4/1 80°C | Product precipitates, filter off and wash with water and dioxane, dissolved in DCM and filtered through through Florisil^{®}, recrystallization from toluene | 8.6g (66%) |
| | | | | m/z = 600 [M+H]⁺ |
| A11 | Sphos Pd(crotyl)Cl 2mol%, Tripotassium phosphate 2.5eq | THF 45°C | product precipitates, washed with water and hexane, recrystallized from DMF | 4.67g (67%) |
| | | | | m/z = 524 [M+H]⁺ |
| A13 | Sphos Pd(crotyl)Cl 6mol%, Tripotassium phosphate 2.5eq | toluene/THF/ H2O 5/2/1 55°C -> 80°C | product precipitates, washed with water, toluene and THF, soxhlet with DCM, product precipitates | 2.6 g (55%) |
| | | | | m/z = 588 [M+H]⁺ |
| A14 | Dichloro[1,1'bis(diphenylphosphi no)ferrocene]palladi um(II) 3mol%, Potassium carbonate 2eq, 2M | toluene/THF/ H₂O 5/2/1 80 °C | product precipitates, washed with water, dissolved in hot chlorobenzene, filtered through SiO2 and allowed to crystallize, filtered and washed with n-hexane | 5.66g (48%) |
| | | | | m/z = 598 [M+H]⁺ |
| A16 | Dichloro[1,1'bis(diphenylphosphi no)ferrocene]palladi um(II) 3mol%, Potassium carbonate 2eq, 2M | toluene/THF/ H2O 5/2/1 80 °C | solvent removed, solid washed with water and extracted by soxhlet with chlorobenzene, product crystallized | 6.55g (46%) |
| | | | | m/z = 498 [M+H]⁺ |

Compound A1 was synthesized as follows:
A flask was flushed with nitrogen and charged with the aldehyde A1-A (structure below) and di(2-pyridyl) ketone in a 1:1 ratio.

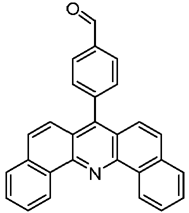

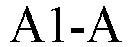

0,1 eq of iodine, 2eq Ammonium Acetate and THF/EtOH 1/1 were added and the mixture was heated to reflux until TLC showed complete consumption of the starting materials. The mixture was then cooled to room temperature and the product was purified in that the solvent was removed, followed by aqueous work-up (CHCl3/ Na2S2O3/ H2O), precipitation from CHCl3/ n-hexane, solid collected, dissolved in hot chlorobenzene, filtered through SiO2 and precipitated by addition of n-hexane. The yield was 3.7 g (19%), m/z = 549 [M+H]+.

Compound A29 was synthesized as follows:
A flask was flushed with nitrogen and charged with the aldehyde A29-A (structure below) and di(pyridin-2-yl)methanone in a 1:1 ratio.

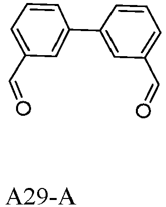

0,1 eq of iodine, 8.8 eq Ammonium Acetate and THF/EtOH 1/1 were added and the mixture was heated to reflux until TLC showed complete consumption of the starting materials.. The mixture was then cooled to room temperature and diluted with MeOH and 2M NaOH. The product was filtered off and recrystallized from toluene. The yield was 3.3 g (52%), m/z = 541 [M+H]+.

### General procedure for fabrication of OLEDs

For bottom emission devices, see Example 1 to 9 and comparative example 1 in Table 3, a 15Ω /cm² glass substrate with 90 nm ITO (available from Corning Co.) was cut to a size of 50 mm x 50 mm x 0.7 mm, ultrasonically washed with isopropyl alcohol for 5 minutes and then with pure water for 5 minutes, and washed again with UV ozone for 30 minutes, to prepare the anode.

Then, 97 vol.-% Biphenyl-4-yl(9,9-diphenyl-9H-fluoren-2-yl)-[4-(9-phenyl-9H-carbazol-3-yl)phenyl]-amine (CAS 1242056-42-3) with 3 vol.-% 2,2',2"-(cyclopropane-1,2,3-triylidene)tris(2-(p-cyanotetrafluorophenyl)acetonitrile) was vacuum deposited on the anode, to form a HIL having a thickness of 10 nm.

Then, Biphenyl-4-yl(9,9-diphenyl-9H-fluoren-2-yl)-[4-(9-phenyl-9H-carbazol-3-yl) phenyl]-amine was vacuum deposited on the HIL, to form a first HTL having a thickness of 118 nm.

Then N,N-bis(4-(dibenzo[b,d]furan-4-yl)phenyl)-[1,1':4',1"-terphenyl]-4-amine (CAS 1198399-61-9) was vacuum deposited on the HTL, to form an electron blocking layer (EBL) having a thickness of 5 nm.

Then 97 vol.-% H09 (Sun Fine Chemicals, Korea) as EML host and 3 vol.-% BD200 (Sun Fine Chemicals, Korea) as fluorescent blue dopant were deposited on the EBL, to form a first blueemitting EML with a thickness of 20 nm.

Then the first hole blocking layer is formed with a thickness of 5 nm by depositing 2-(3'-(9,9-dimethyl-9H-fluoren-2-yl)-[1,1'-biphenyl]-3-yl)-4,6-diphenyl-1,3,5-triazine on the emission layer.

Then, the electron transporting layer having a thickness of 25 nm is formed on the hole blocking layer by depositing 4'-(4-(4-(4,6-diphenyl-1,3,5-triazin-2-yl)phenyl)naphthalen-1-yl)-[1,1'-biphenyl]-4-carbonitrile. The electron transport layer (ETL) comprises 50 wt.-% matrix compound and 50 wt.-% of LiQ.

Then the n-CGL was formed on ETL with a thickness of 15 nm. The composition of the n-CGL can be taken from Table 2.

Then the p-CGL was formed on n-CGL with a thickness of 10 nm by depositing Biphenyl-4-yl(9,9-diphenyl-9H-fluoren-2-yl)-[4-(9-phenyl-9H-carbazol-3-yl)phenyl]-amine (CAS 1242056-42-3) with 2,2',2"-(cyclopropane-1,2,3-triylidene)tris(2-(p-cyanotetrafluorophenyl)acetonitrile).

Then, Biphenyl-4-yl(9,9-diphenyl-9H-fluoren-2-yl)-[4-(9-phenyl-9H-carbazol-3-yl) phenyl]-amine was vacuum deposited on the p-CGL, to form a second HTL having a thickness of 10 nm.

A1 is evaporated at a rate of 0.01 to 1 Å/s at 10⁻⁷ mbar to form a cathode with a thickness of 100 nm.

The OLED stack is protected from ambient conditions by encapsulation of the device with a glass slide. Thereby, a cavity is formed, which includes a getter material for further protection.

To assess the performance of the inventive examples compared to the prior art, the current efficiency is measured at 20°C. The current-voltage characteristic is determined using a Keithley 2635 source measure unit, by sourcing a voltage in V and measuring the current in mA flowing through the device under test. The voltage applied to the device is varied in steps of 0.1V in the range between 0V and 10V. Likewise, the luminance-voltage characteristics and CIE coordinates are determined by measuring the luminance in cd/m² using an Instrument Systems CAS-140CT array spectrometer (calibrated by Deutsche Akkreditierungsstelle (DAkkS)) for each of the voltage values. The cd/A efficiency at 10 mA/cm2 is determined by interpolating the luminance-voltage and current-voltage characteristics, respectively.

Lifetime LT of the device is measured at ambient conditions (20°C) and 30 mA/cm², using a Keithley 2400 sourcemeter, and recorded in hours.

The brightness of the device is measured using a calibrated photo diode. The lifetime LT is defined as the time till the brightness of the device is reduced to 97 % of its initial value.

### Technical Effect of the invention

In order to investigate the usefulness of the inventive compound preferred materials were tested in model top-emission blue OLEDs prepared as described above.

As a comparative example the following compound was used:

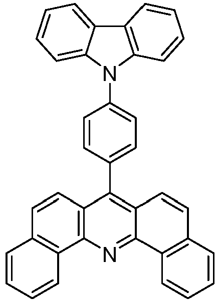

In the following organic electronic devices were prepared according to several examples of the present invention and their properties were juxtaposed with a device according to a comparative example. The results are shown in the following Table 3:

**Table 3: Properties of several organic electronic devices**

| Matrix compound | Concentra tion of matrix compound (vol.-%) | Metal dopant | Concen tration of metal dopant (vol.-%) | Thickness organic semiconductor layer (nm) | Operating voltage at 10 mA/cm ² (V) | cd/A efficien cy at 10 mA/cm ² (cd/A) | lm/W efficien cy at 10 mA/cm ² (lm/W) | EQE at 10 mA/c m² (%) |
|---|---|---|---|---|---|---|---|---|
| Compara tive | 99 | Li | 1 | 15 | 12 | < 0.1 | < 0.1 | < 0.1 |
| A2 | 99 | Li | 1 | 15 | 4.8 | 6.4 | 4.4 | 5.2 |
| A3 | 99 | Li | 1 | 15 | 4.9 | 6.4 | 4.1 | 5.2 |
| A8 | 99 | Li | 1 | 15 | 5.2 | 5.8 | 3.7 | 5.7 |
| A11 | 99 | Li | 1 | 15 | 5 | 6.6 | 4.1 | 5.2 |
| A16 | 99 | Li | 1 | 15 | 5.4 | 6 | 3.7 | 5.7 |
| A14 | 99 | Li | 1 | 15 | 5.2 | 6.4 | 4.1 | 5.3 |
| A14 | 98 | Li | 2 | 15 | 5.1 | 6.3 | 4.1 | 5.2 |
| A2 | 99 | Yb | 1 | 15 | 5.2 | 6.5 | 4.1 | 5.5 |
| A3 | 99 | Yb | 1 | 15 | 5.4 | 6.7 | 4.3 | 5.2 |
| A11 | 99 | Yb | 1 | 15 | 5.5 | 6.5 | 4.1 | 5.7 |
| A11 | 99 | Yb | 1 | 15 | 5.3 | 6.2 | 3.9 | 5.3 |

The results show that in comparison with state-of-art reference, the compounds according to invention show a clearly enhanced performance, especially concerning efficiency and EQE. Accordingly, the foregoing description is by way of example only and is not intended as limiting. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. The invention's scope is defined in the following claims.

Furthermore, reference signs used in the description and claims do not limit the scope of the invention as claimed.

## Claims

1. An organic electronic device comprising an anode (120), a cathode (190), at least one photoactive layer (125) and an organic semiconductor layer (160), wherein the organic semiconductor layer is arranged between the at least one photoactive layer and the cathode; and wherein the organic semiconductor layer comprises a compound of Formula (1): 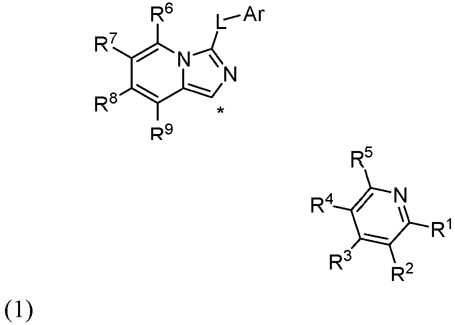 wherein
one of R¹ to R⁵ is a single bond to the 3-position (marked as "*") of the 2-azaindolizine moiety,
the further R¹ to R⁵ and R⁶ to R⁹ are independently selected from H, D, substituted or unsubstituted C₆ to C₁₈ aryl, substituted or unsubstituted C₃ to C₂₀ heteroaryl, C₁ to C₁₆ alkyl, C₁ to C₁₆ alkoxy, C₃ to C₁₆ branched alkyl, C₃ to C₁₆ cyclic alkyl, C₃ to C₁₆ branched alkoxy, C₃ to C₁₆ cyclic alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy, PX¹(R¹⁰)₂, F or CN, and/or wherein any two of adjacent R¹-R⁹ can be suitably substituted and linked together to form an unsubstituted or an C₆ to C₁₈ aryl-, C₃ to C₂₀ heteroaryl-, or C₁ to C₁₆ alkyl-substituted aromatic or heteroaromatic ring;
L is selected from a substituted or unsubstituted C₆ to C₂₄ arylene group or a substituted or unsubstituted C₂ to C₂₄ heteroarylene group;
Ar is selected from any of the following moieties D1 to D76: 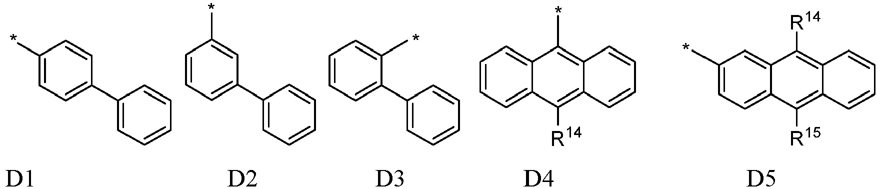 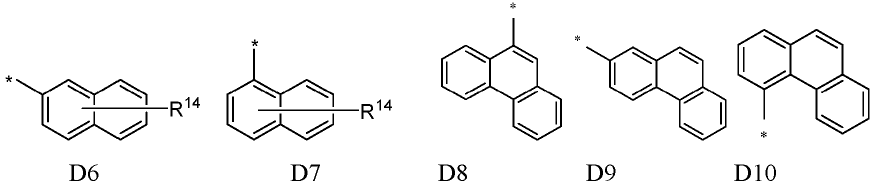 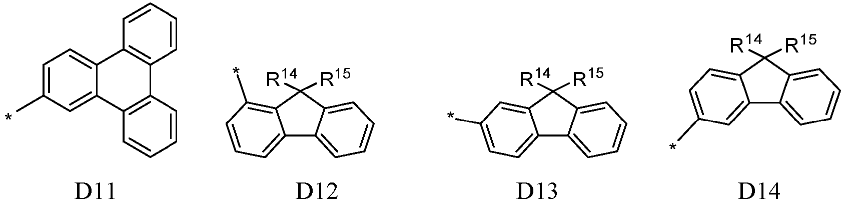 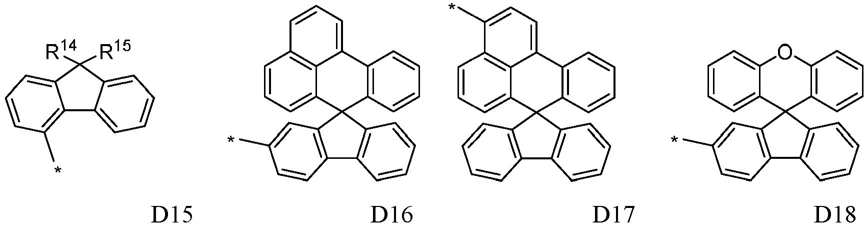 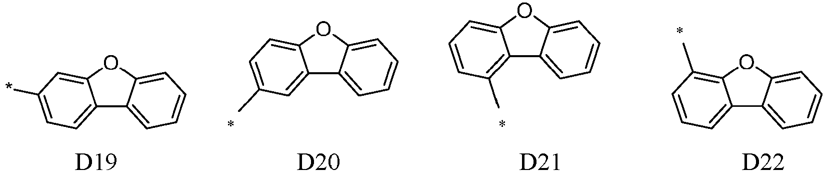 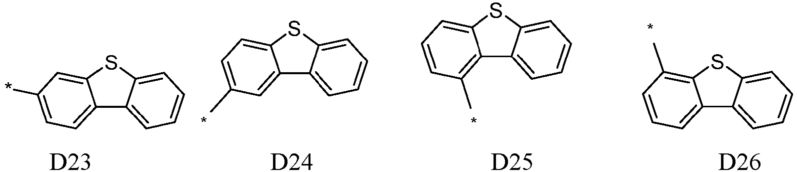 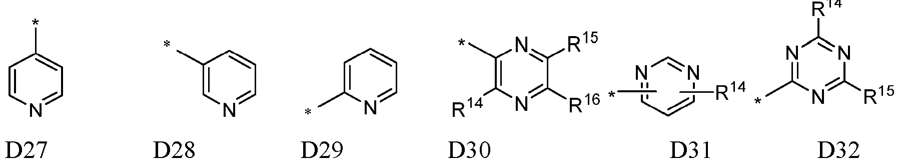 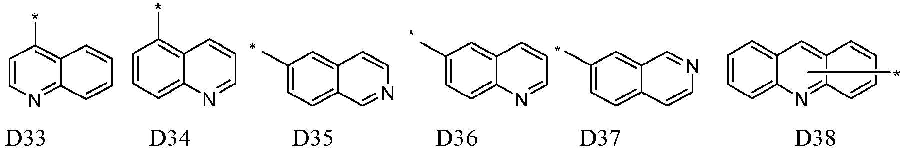 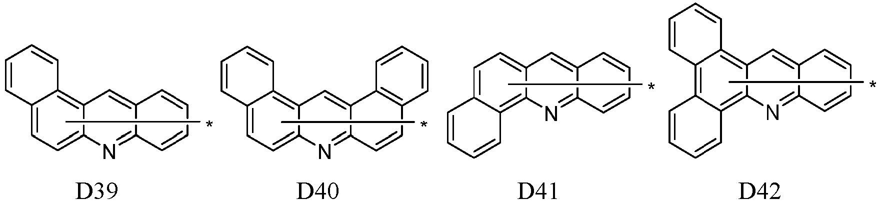 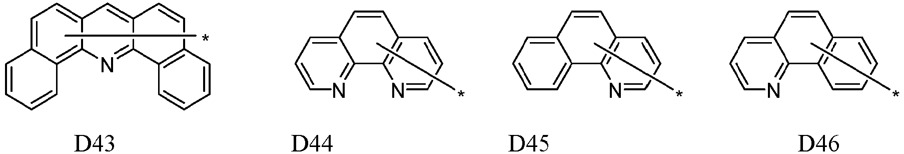 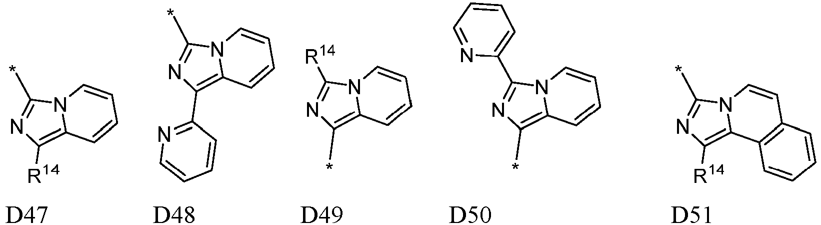 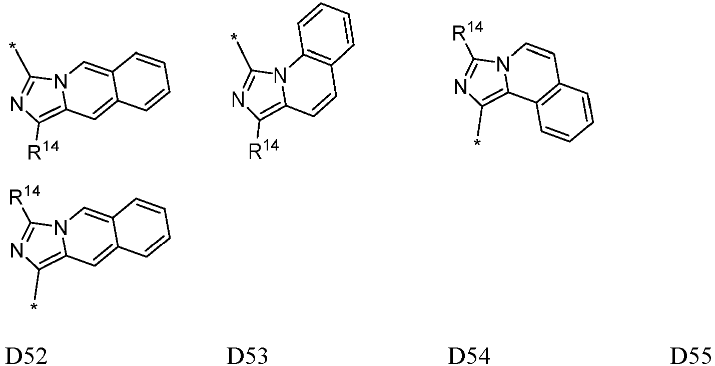 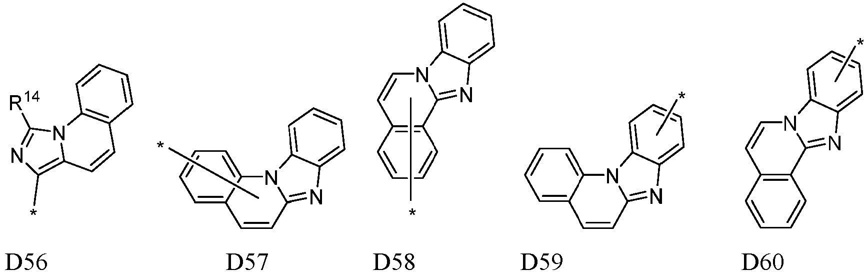 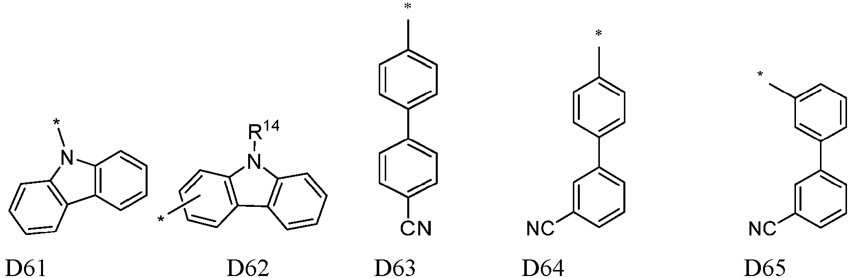 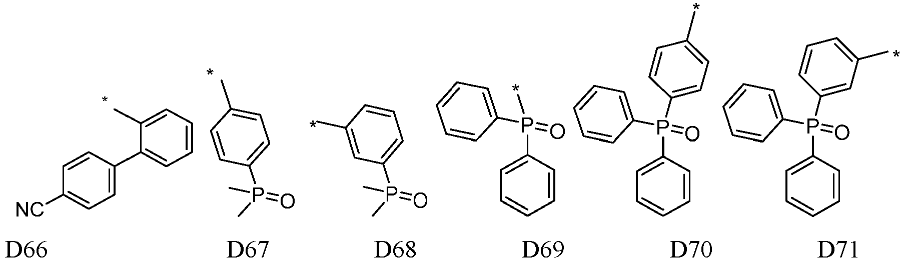 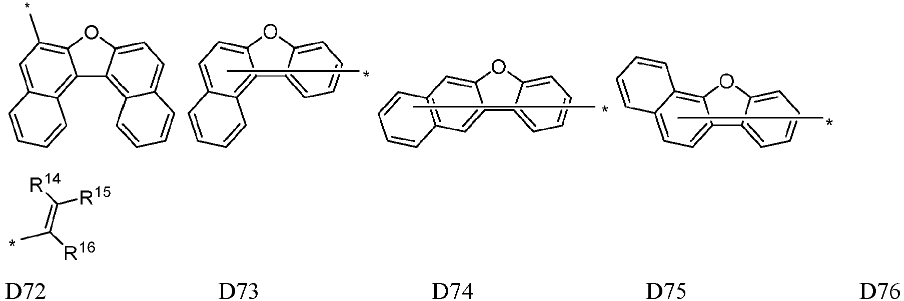 wherein
R¹⁴, R¹⁵ and R¹⁶ are independently selected from H, C₁ to C₁₆ alkyl, C₁ to C₁₆ alkoxy, C₆ to C₁₈ aryl, C₃ to C₂₀ heteroaryl, perfluorinated C₁ to C₁₆ alkyl, perfluorinated C₁ to C₁₆ alkoxy, wherein R¹⁴ and R¹⁵ may be linked via a single bond or a heteroatom to form a ring.
wherein the substituents of L and Ar are independently selected from:
H, D, C₆ to C₁₈ aryl, C₃ to C₂₀ heteroaryl, C₁ to C₁₆ alkyl, C₁ to C₁₆ alkoxy, C₃ to C₁₆ branched alkyl, C₃ to C₁₆ cyclic alkyl, C₃ to C₁₆ branched alkoxy, C₃ to C₁₆ cyclic alkoxy,
partially or perfluorinated C₁ to C₁₆ alkyl, partially or perfluorinated C₁ to C₁₆ alkoxy,
partially or perdeuterated C₁ to C₁₆ alkyl, partially or perdeuterated C₁ to C₁₆ alkoxy, F, CN or PX¹(R¹⁰)₂, wherein the substituents may be linked via a single bond or a heteroatom to form a ring,
wherein R¹⁰ is independently selected from C₆ to C₁₂ aryl, C₃ to C₁₂ heteroaryl, C₁ to C₁₆ alkyl, C₁ to C₁₆ alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy;
and X¹ is selected from O, S or Se, preferably O and wherein the compound of formula (I) does not include the following compounds:

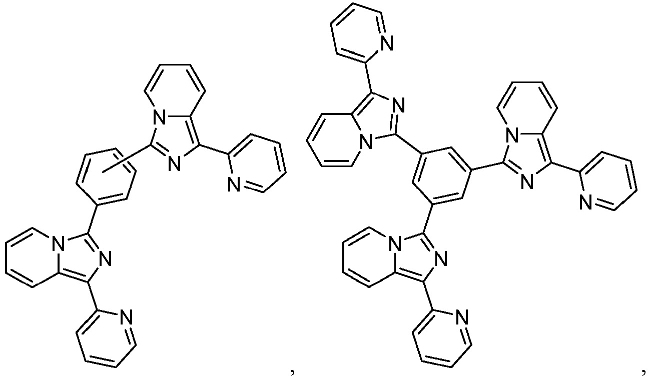

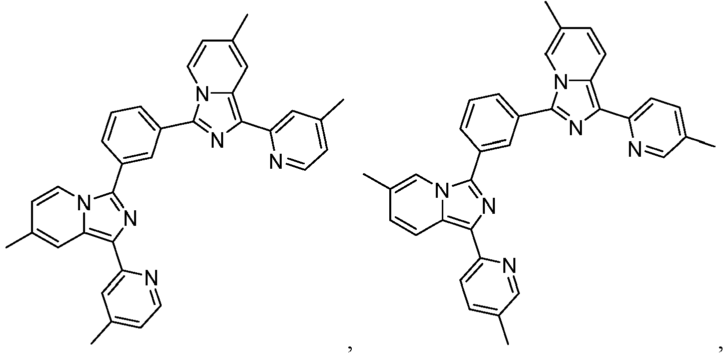

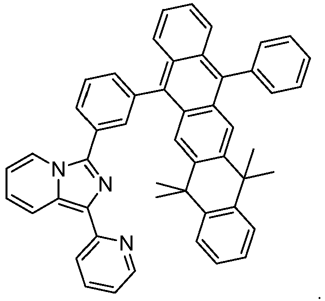

2. The organic electronic device of claim 1, whereby the substituents of the further R¹ to R⁵ and R⁶ to R⁹ which do not form a single bond to the 3-position of the 2-azaindolizine moiety are independently selected from D, -CH=, C₆ to C₁₈ aryl, C₃ to C₂₀ heteroaryl, C₁ to C₁₆ alkyl, C₁ to C₁₆ alkoxy, C₃ to C₁₆ branched alkyl, C₃ to C₁₆ cyclic alkyl, C₃ to C₁₆ branched alkoxy, C₃ to C₁₆ cyclic alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy, PX¹(R¹⁰)₂, F or CN.

3. The organic electronic device of claim 1 or 2 whereby the compound has the following formula (1a):

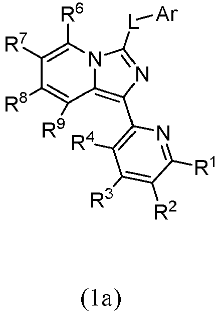

4. The organic electronic device of any of the claims 1 to 3 where the organic layer and/or the compound of formula (1) or (1a) are non-emissive.

5. The organic electronic device of any of the claims 1 to 4, whereby the R¹ to R⁹ which do not form a single bond to the 3-position of the 2-azaindolizine moiety are independently selected from H, -CH=, C₁ to C₄ alkyl, F or CN.

6. The organic electronic device of any of the claims 1 to 5, whereby the compound of formula (1) is selected from one of the following formulas (2a) to (2f): 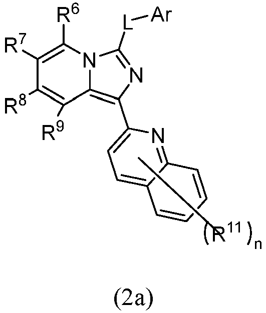 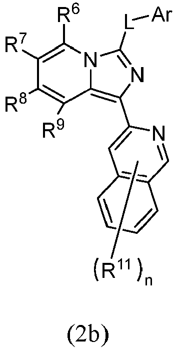 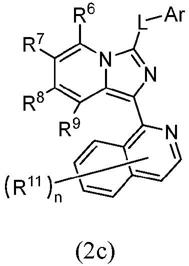 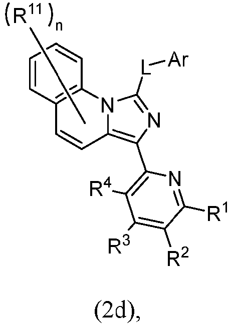 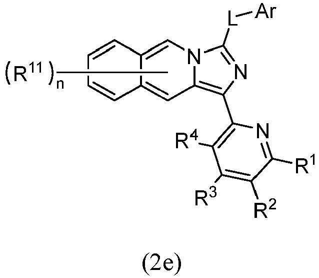 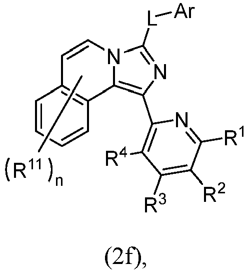 wherein R¹¹ is independently selected from D, C₆ to C₁₈ aryl, C₃ to C₂₀ heteroaryl, C₁ to C₁₆ alkyl, C₁ to C₁₆ alkoxy, C₃ to C₁₆ branched alkyl, C₃ to C₁₆ cyclic alkyl, C₃ to C₁₆ branched alkoxy, C₃ to C₁₆ cyclic alkoxy, partially or perfluorinated C₁ to C₁₆ alkyl, partially or perfluorinated C₁ to C₁₆ alkoxy, partially or perdeuterated C₁ to C₁₆ alkyl, partially or perdeuterated C₁ to C₁₆ alkoxy, PX¹(R¹⁰)₂, F or CN; and
n is an integer from 0 to 4.

7. The organic electronic device of any of the claims 1 to 6, whereby the moiety L is selected any of the following moieties E1 to E26: 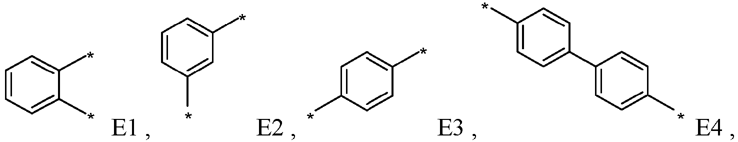 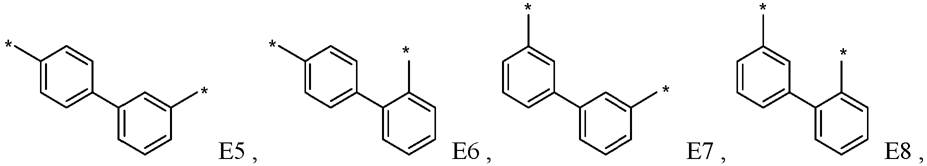 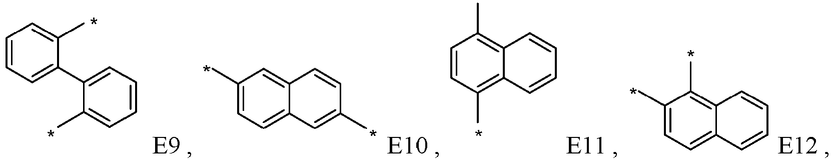 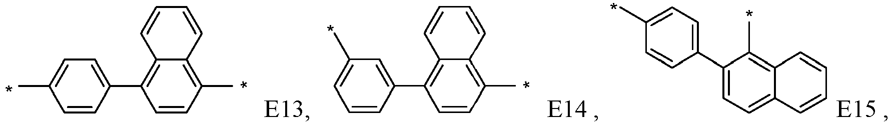 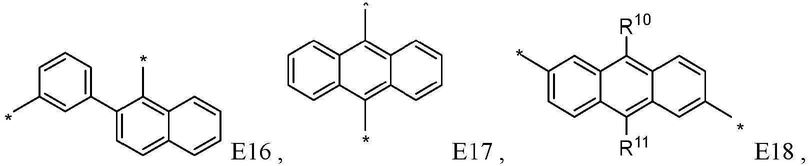 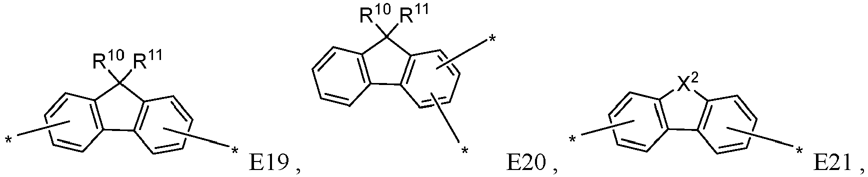 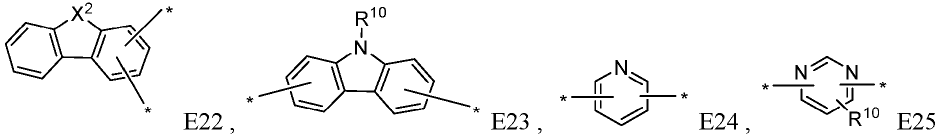 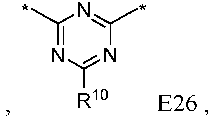 wherein
X² is selected from O or S, preferably O;
R¹⁰ and R¹¹ are independently selected from H, C₁ to C₁₆ alkyl, C₁ to C₁₆ alkoxy, C₆ to C₁₈ aryl, C₃ to C₂₀ heteroaryl, perfluorinated C₁ to C₁₆ alkyl, perfluorinated C₁ to C₁₆ alkoxy.

8. The organic electronic device of any of the claims 1 to 7, whereby the organic semiconductor layer comprises a redox n-dopant.

9. The organic electronic device of any of the claims 1 to 8, whereby the organic semiconductor layer comprises a metal.

10. The organic electronic device of any of the claims 1 to 9, whereby the organic semiconductor layer comprises a metal selected from alkali metals, alkaline earth metals, rare earth metals and metals of the first transition period Ti, V, Cr and Mn.

11. The electronic organic electronic device of any of the claims 1 to 10, whereby the at least one photoactive layer is a light-emitting layer.

12. The electronic organic device of any of the claims 1 to 11, whereby the electronic organic device is an electroluminescent device, preferably an organic light emitting diode.

13. A display device comprising an organic electronic device according to any of the claims 1 to 12.

14. The compound of formula (1) as defined in claim 1.

## Patentansprüche

1. Organische elektronische Vorrichtung, umfassend eine Anode (120), eine Kathode (190), mindestens eine photoaktive Schicht (125) und eine organische Halbleiterschicht (160), wobei die organische Halbleiterschicht zwischen der mindestens einen photoaktiven Schicht und der Kathode angeordnet ist und wobei die organische Halbleiterschicht eine Verbindung der Formel (1) umfasst: 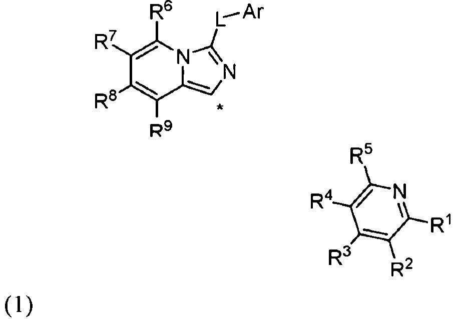 wobei
eines von R¹ bis R⁵ für eine Einfachbindung an die 3-Position (als "*" markiert) der 2-Azaindolizin-Gruppierung steht,
die weiteren R¹ bis R⁵ und R⁶ bis R⁹ unabhängig aus H, D, substituiertem oder unsubstituiertem C₆- bis C₁₈-Aryl, substituiertem oder unsubstituiertem C₃- bis C₂₀-Heteroaryl, C₁- bis C₁₆-Alkyl, C₁- bis C₁₆-Alkoxy, verzweigtem C₃- bis C₁₆-Alkyl, cyclischem C₃- bis C₁₆-Alkyl, verzweigtem C₃- bis C₁₆-Alkoxy, cyclischem C₃- bis C₁₆-Alkoxy, teil- oder perfluoriertem C₁- bis C₁₆-Alkyl, teil- oder perfluoriertem C₁- bis C₁₆-Alkoxy, teil- oder perdeuteriertem C₁- bis C₁₆-Alkyl, teil- oder perdeuteriertem C₁- bis C₁₆-Alkoxy, PX¹(R¹⁰)₂, F oder CN ausgewählt sind und/oder wobei beliebige zwei benachbarte R¹-R⁹ geeignet substituiert und miteinander zu einem unsubstituierten oder einem C₆- bis C₁₈-aryl-, C₃- bis C₂₀-heteroaryl- oder C₁- bis C₁₆-alkyl-substituierten aromatischen oder heteroaromatischen Ring miteinander verknüpft sein können;
L aus einer substituierten oder unsubstituierten C₆-C₂₄-Arylengruppe oder einer substituierten oder unsubstituierten C₂-C₂₄-Heteroarylengruppe ausgewählt ist;
Ar aus einer der folgenden Gruppierungen D1 bis D76 ausgewählt ist: 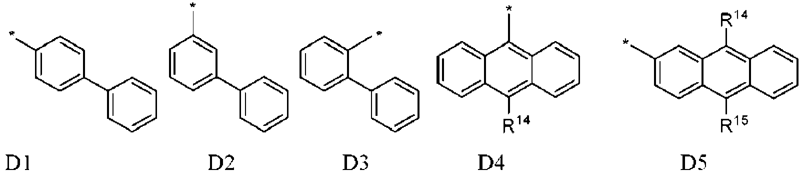 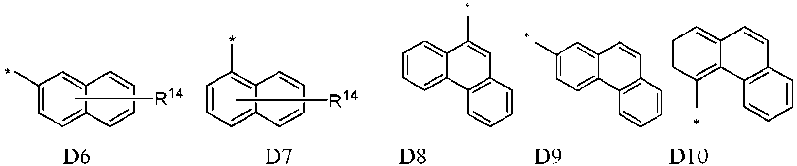 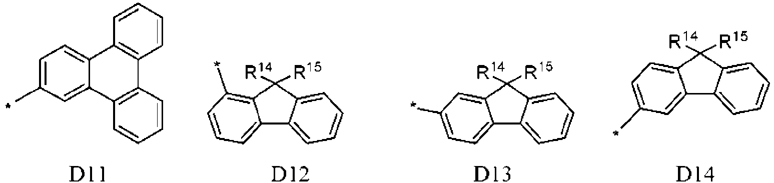 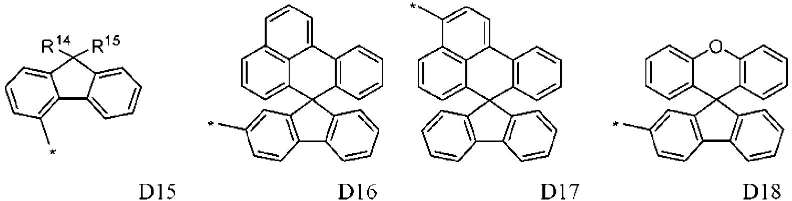 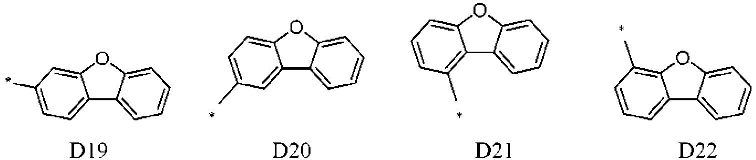 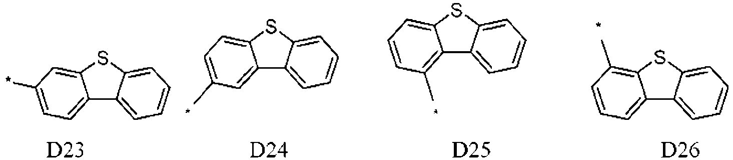 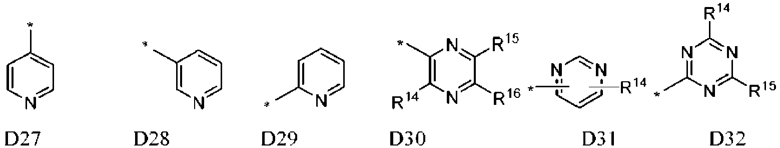 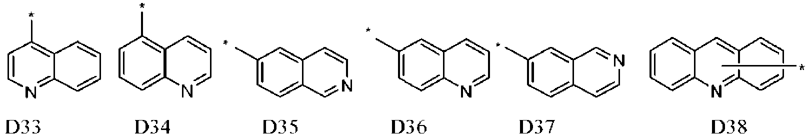 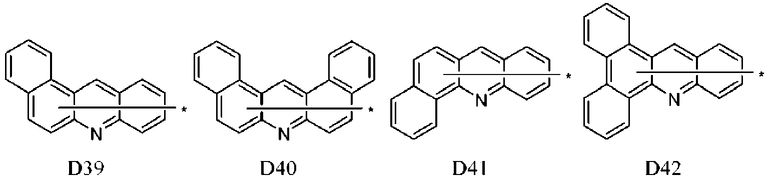 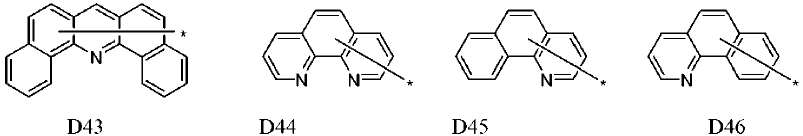 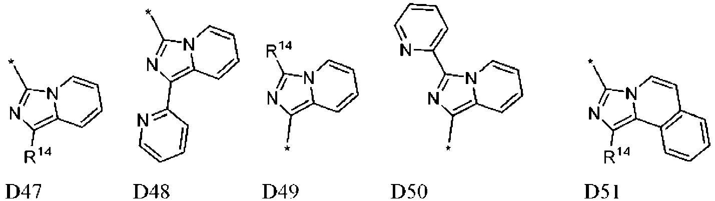 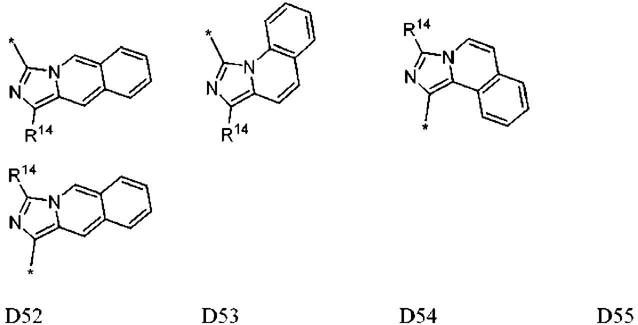 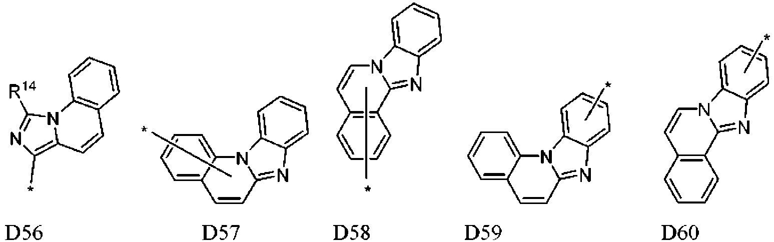 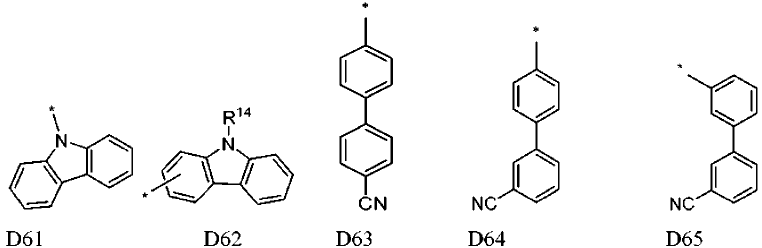 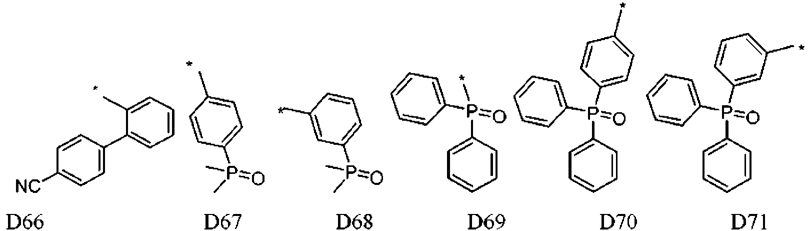 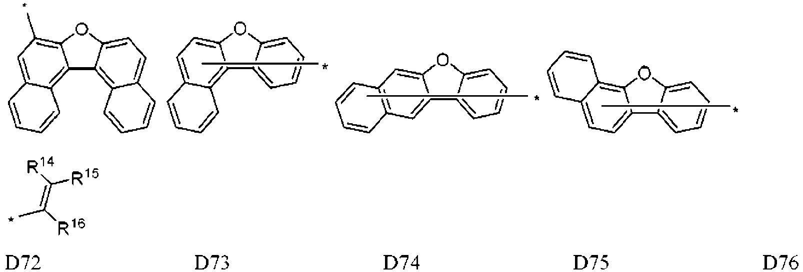 wobei R¹⁴, R¹⁵ und R¹⁶ unabhängig aus H, C₁- bis C₁₆-Alkyl, C₁-bis C₁₆-Alkoxy, C₆- bis C₁₈-Aryl, C₃- bis C₂₀-Heteroaryl, perfluoriertem C₁- bis C₁₆-Alkyl, perfluoriertem C₁- bis C₁₆-Alkoxy ausgewählt sind, wobei R¹⁴ und R¹⁵ über eine Einfachbindung oder ein Heteroatom zu einem Ring verknüpft sein können;
wobei die Substituenten von L und Ar unabhängig ausgewählt sind aus:
H, D, C₆- bis C₁₈-Aryl, C₃- bis C₂₀-Heteroaryl, C₁- bis C₁₆-Alkyl, C₁- bis C₁₆-Alkoxy, verzweigtem C₃- bis C₁₆-Alkyl, cyclischem C₃- bis C₁₆-Alkyl, verzweigtem C₃- bis C₁₆-Alkoxy, cyclischem C₃- bis C₁₆-Alkoxy, teil- oder perfluoriertem C₁- bis C₁₆-Alkyl, teil- oder perfluoriertem C₁- bis C₁₆-Alkoxy, teil- oder perdeuteriertem C₁- bis C₁₆-Alkyl, teil- oder perdeuteriertem C₁- bis C₁₆-Alkoxy, F, CN oder PX¹(R¹⁰)₂, wobei die Substituenten über eine Einfachbindung oder ein Heteroatom zu einem Ring verknüpft sein können,
wobei R¹⁰ unabhängig aus C₆- bis C₁₂-Aryl, C₃- bis C₁₂-Heteroaryl, C₁- bis C₁₆-Alkyl, C₁- bis C₁₆-Alkoxy, teil-oder perfluoriertem C₁- bis C₁₆-Alkyl, teil- oder perfluoriertem C₁- bis C₁₆-Alkoxy, teil- oder perdeuteriertem C₁- bis C₁₆-Alkyl, teil- oder perdeuteriertem C₁- bis C₁₆-Alkoxy ausgewählt ist;
und X¹ aus O, S oder Se, vorzugsweise O, ausgewählt ist und wobei die Verbindung der Formel (I) die folgenden Verbindungen nicht umfasst:

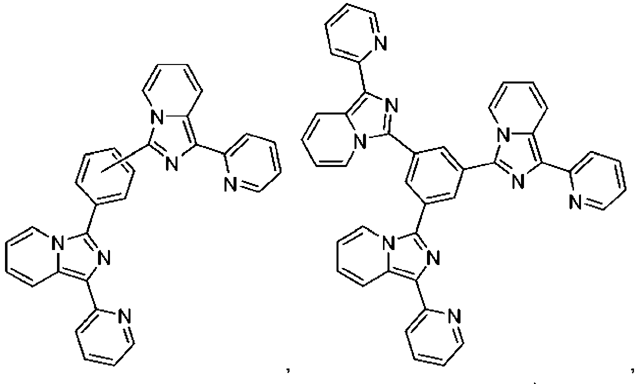

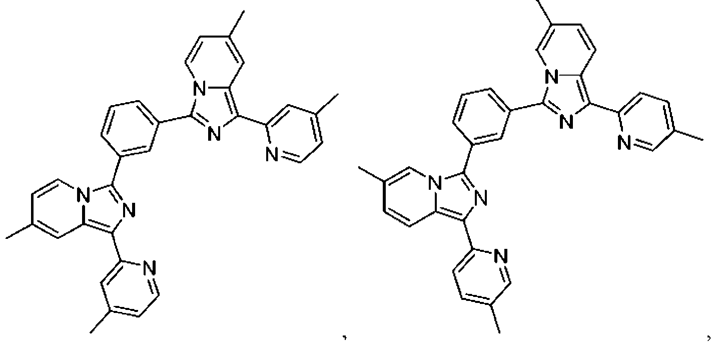

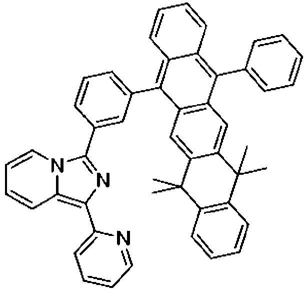

2. Organische elektronische Vorrichtung nach Anspruch 1, wobei die Substituenten der weiteren R¹ bis R⁵ und R⁶ bis R⁹, die keine Einfachbindung an die 3-Position der 2-Azaindolizin-Gruppierung bilden, unabhängig aus D, -CH=, C₆- bis C₁₈-Aryl, C₃- bis C₂₀-Heteroaryl, C₁- bis C₁₆-Alkyl, C₁- bis C₁₆-Alkoxy, verzweigtem C₃- bis C₁₆-Alkyl, cyclischem C₃- bis C₁₆-Alkyl, verzweigtem C₃- bis C₁₆-Alkoxy, cyclischem C₃- bis C₁₆-Alkoxy, teil- oder perfluoriertem C₁- bis C₁₆-Alkyl, teil- oder perfluoriertem C₁- bis C₁₆-Alkoxy, teil- oder perdeuteriertem C₁- bis C₁₆-Alkyl, teil- oder perdeuteriertem C₁- bis C₁₆-Alkoxy, PX¹ (R¹⁰) ₂, F oder CN ausgewählt sind.

3. Organische elektronische Vorrichtung nach Anspruch 1 oder 2, wobei die Verbindung die folgende Formel (1a) aufweist:

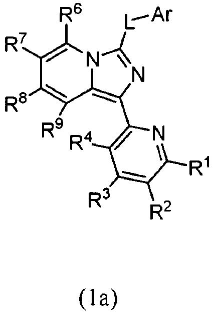

4. Organische elektronische Vorrichtung nach einem der Ansprüche 1 bis 3, wobei die organische Schicht und/oder die Verbindung der Formel (1) oder (1a) nichtemissiv sind.

5. Organische elektronische Vorrichtung nach einem der Ansprüche 1 bis 4, wobei die R¹ bis R⁹, die keine Einfachbindung an die 3-Position der 2-Azaindolizin-Gruppierung bilden, unabhängig aus H, -CH=, C₁- bis C₄-Alkyl, F oder CN ausgewählt sind.

6. Organische elektronische Vorrichtung nach einem der Ansprüche 1 bis 5, wobei die Verbindung der Formel (1) aus den folgenden Formeln (2a) bis (2f) ausgewählt ist: 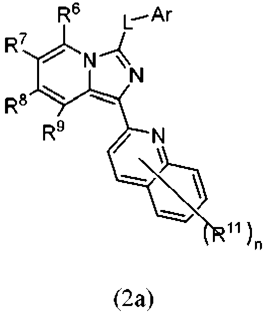 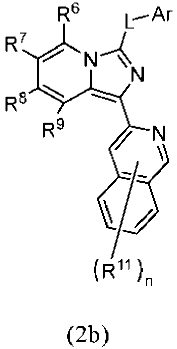 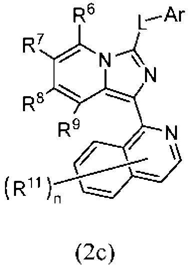 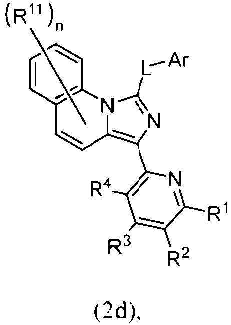 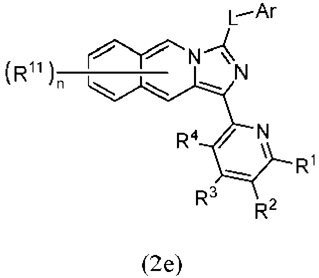 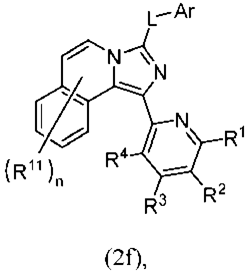 wobei R¹¹ unabhängig aus D, C₆- bis C₁₈-Aryl, C₃- bis C₂₀-Heteroaryl, C₁- bis C₁₆-Alkyl, C₁- bis C₁₆-Alkoxy, verzweigtem C₃- bis C₁₆-Alkyl, cyclischem C₃- bis C₁₆-Alkyl, verzweigtem C₃- bis C₁₆-Alkoxy, cyclischem C₃- bis C₁₆-Alkoxy, teil- oder perfluoriertem C₁- bis C₁₆-Alkyl, teil- oder perfluoriertem C₁- bis C₁₆-Alkoxy, teil- oder perdeuteriertem C₁- bis C₁₆-Alkyl, teil- oder perdeuteriertem C₁- bis C₁₆-Alkoxy, PX¹(R¹⁰)₂, F oder CN ausgewählt ist; und
n für eine ganze Zahl von 0 bis 4 steht.

7. Organische elektronische Vorrichtung nach einem der Ansprüche 1 bis 6, wobei die Gruppierung L aus einer der folgenden Gruppierungen E1 bis E26 ausgewählt ist: 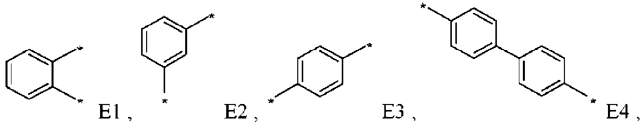 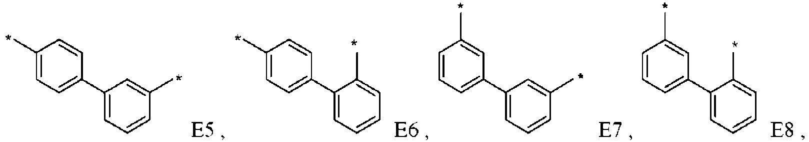 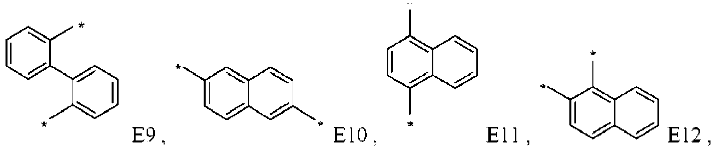 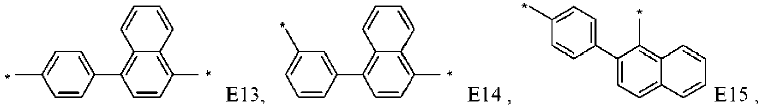 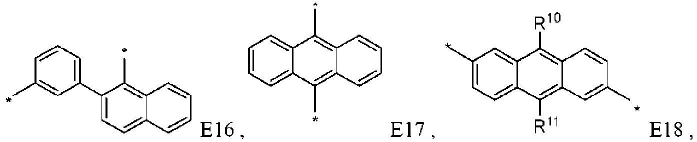 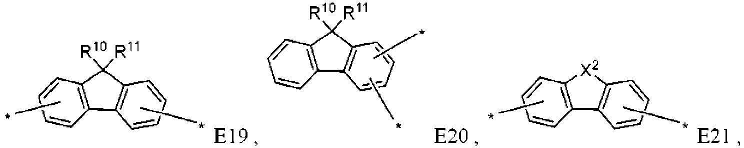 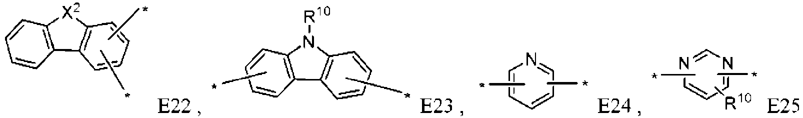 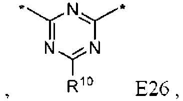 wobei
X² aus O oder S, vorzugsweise O, ausgewählt ist;
R¹⁰ und R¹¹ unabhängig aus H, C₁- bis C₁₆-Alkyl, C₁- bis C₁₆-Alkoxy, C₆- bis C₁₈-Aryl, C₃- bis C₂₀-Heteroaryl, perfluoriertem C₁- bis C₁₆-Alkyl, perfluoriertem- C₁ bis C₁₆-Alkoxy ausgewählt ist.

8. Organische elektronische Vorrichtung nach einem der Ansprüche 1 bis 7, wobei die organische Halbleiterschicht einen Redox-n-Dotierstoff umfasst.

9. Organische elektronische Vorrichtung nach einem der Ansprüche 1 bis 8, wobei die eine Halbleiterschicht ein Metall umfasst.

10. Organische elektronische Vorrichtung nach einem der Ansprüche 1 bis 9, wobei die organische Halbleiterschicht ein Metall umfasst, das aus Alkalimetallen, Erdalkalimetallen, Seltenerdmetallen und Metallen der ersten Übergangsperiode Ti, V, Cr und Mn ausgewählt ist.

11. Organische elektronische Vorrichtung nach einem der Ansprüche 1 bis 10, wobei es sich bei der mindestens einen photoaktiven Schicht um eine lichtemittierende Schicht handelt.

12. Elektronische organische Vorrichtung nach einem der Ansprüche 1 bis 11, wobei es sich bei der elektronischen organischen Vorrichtung um eine Elektrolumineszenzvorrichtung, vorzugsweise eine organische Leuchtdiode, handelt.

13. Anzeigevorrichtung, umfassend eine organische elektronische Vorrichtung gemäß einem der Ansprüche 1 bis 12.

14. Verbindung der Formel (1) gemäß Anspruch 1.

## Revendications

1. Dispositif électronique organique comprenant une anode (120), une cathode (190), au moins une couche photoactive (125) et une couche de semi-conducteur organique (160), la couche de semi-conducteur organique étant agencée entre l'au moins une couche photoactive et la cathode ; et la couche de semi-conducteur organique comprenant un composé de Formule (1) : 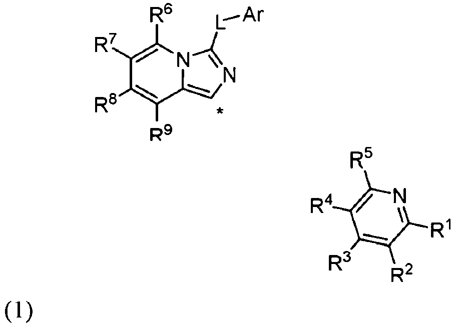 l'un parmi R¹ à R⁵ étant une simple liaison à la position 3 (marquée comme « * ») du groupement 2-azaindolizine, les autres R¹ à R⁵ et R⁶ à R⁹ étant indépendamment choisis parmi H, D, C₆ à C₁₈ aryle substitué ou non substitué, C₃ à C₂₀ hétéroaryle substitué ou non substitué, C₁ à C₁₆ alkyle, C₁ à C₁₆ alcoxy, C₃ à C₁₆ alkyle ramifié, C₃ à C₁₆ alkyle cyclique, C₃ à C₁₆ alcoxy ramifié, C₃ à C₁₆ alcoxy cyclique, C₁ à C₁₆ alkyle partiellement ou perfluoré, C₁ à C₁₆ alcoxy partiellement ou perfluoré, C₁ à C₁₆ alkyle partiellement ou perdeutéré, C₁ à C₁₆ alcoxy partiellement ou perdeutéré, PX¹(R¹⁰)₂, F ou CN, et/ou deux quelconques parmi des R¹-R⁹ adjacents pouvant être de manière appropriée substitués et liés ensemble pour former un cycle aromatique ou hétéroaromatique non substitué ou substitué par C₆ à C₁₈ aryle, C₃ à C₂₀ hétéroaryle ou C₁ à C₁₆ alkyle ;
L étant choisi parmi un groupe C₆ à C₂₄ arylène substitué ou non substitué ou un groupe C₂ à C₂₄ hétéroarylène substitué ou non substitué ;
Ar étant choisi parmi l'un quelconque des groupements suivants D1 à D76 : 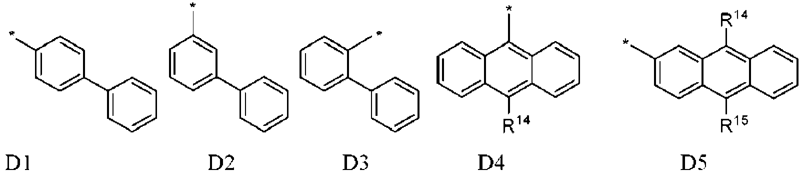 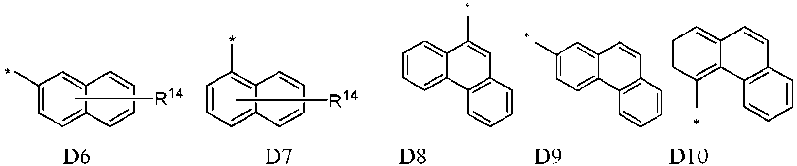 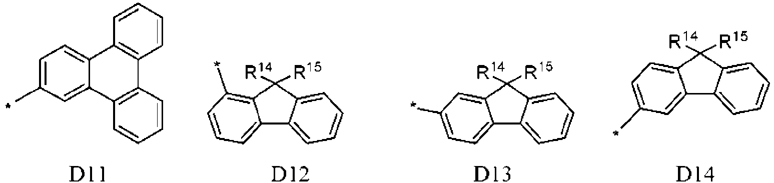 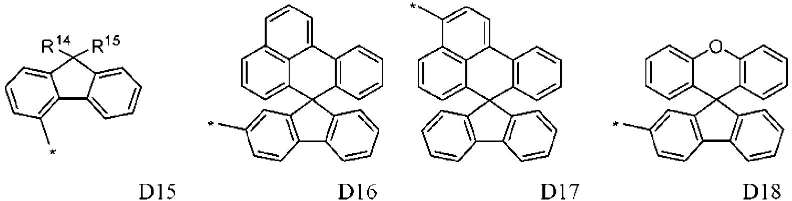 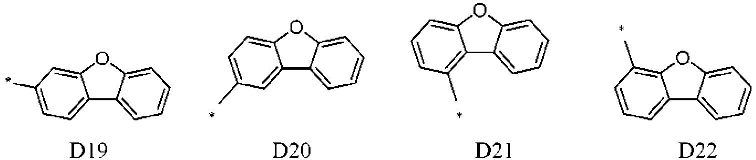 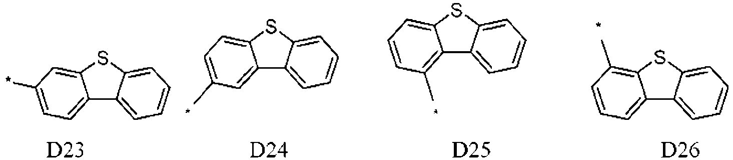 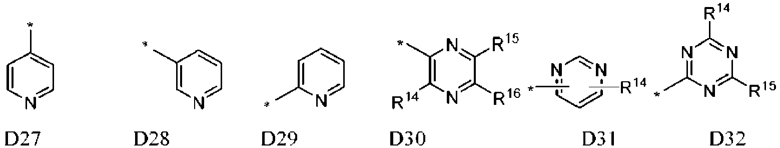 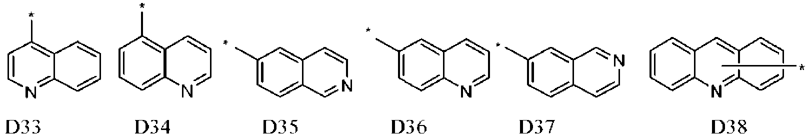 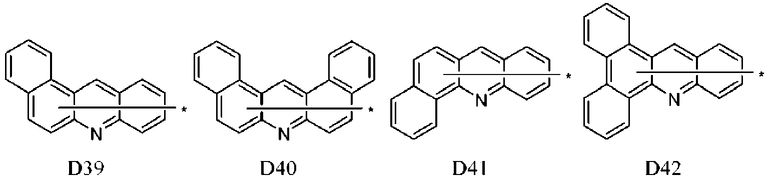 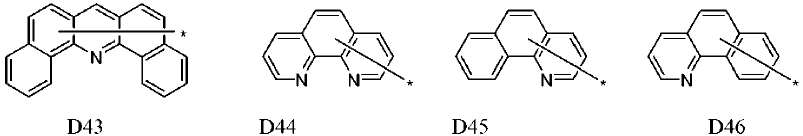 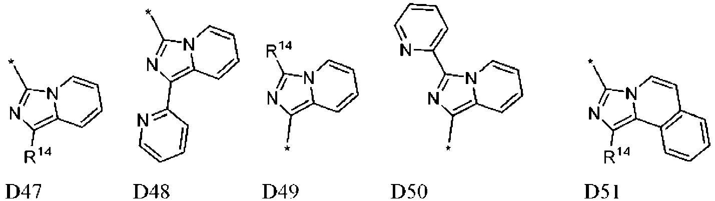 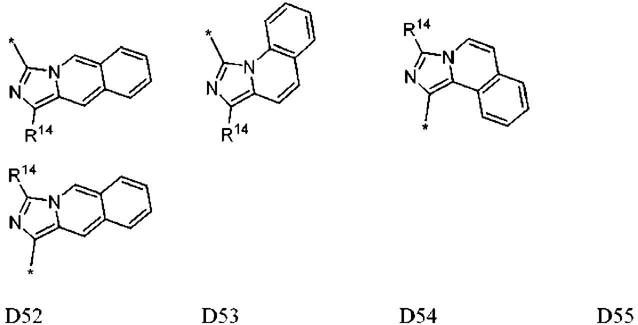 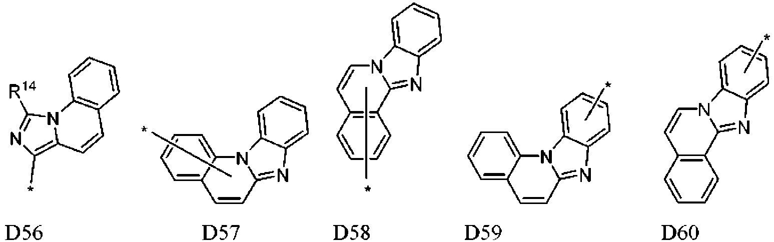 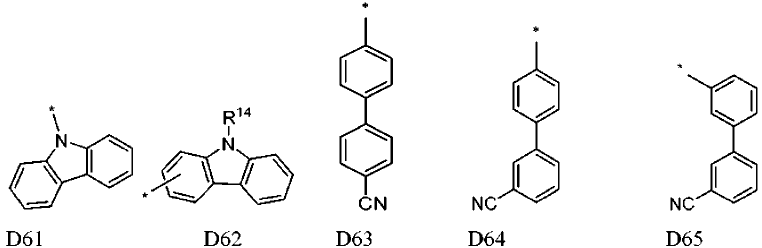 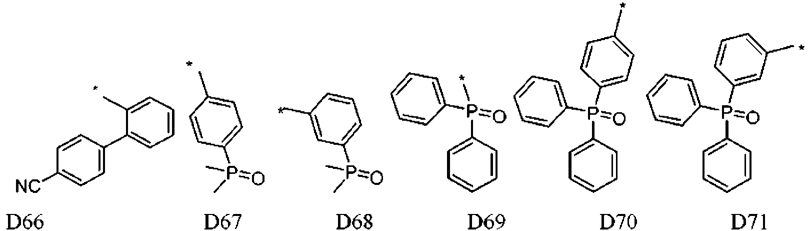 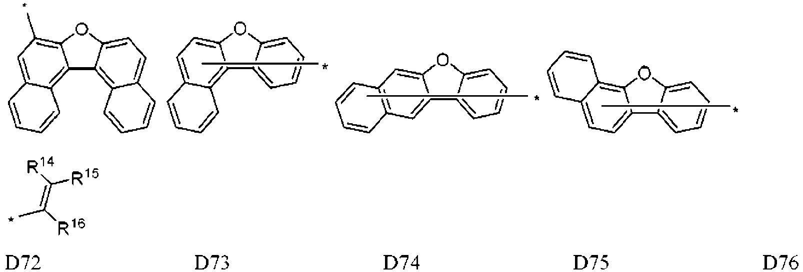
R¹⁴, R¹⁵ et R¹⁶ étant indépendamment choisis parmi H, C₁ à C₁₆ alkyle, C₁ à C₁₆ alcoxy, C₆ à C₁₈ aryle, C₃ à C₂₀ hétéroaryle, C₁ à C₁₆ alkyle perfluoré, C₁ à C₁₆ alcoxy perfluoré, R¹⁴ et R¹⁵ pouvant être liés via une simple liaison ou un hétéroatome pour former un cycle.
les substituants de L et Ar étant indépendamment choisis parmi :
H, D, C₆ à C₁₈ aryle, C₃ à C₂₀ hétéroaryle, C₁ à C₁₆ alkyle, C₁ à C₁₆ alcoxy, C₃ à C₁₆ alkyle ramifié, C₃ à C₁₆ alkyle cyclique, C₃ à C₁₆ alcoxy ramifié, C₃ à C₁₆ alcoxy cyclique, C₁ à C₁₆ alkyle partiellement ou perfluoré, C₁ à C₁₆ alcoxy partiellement ou perfluoré, C₁ à C₁₆ alkyle partiellement ou perdeutéré, C₁ à C₁₆ alcoxy partiellement ou perdeutéré, F, CN ou PX¹(R¹⁰)₂, les substituants pouvant être liés via une simple liaison ou un hétéroatome pour former un cycle,
R¹⁰ étant indépendamment choisi parmi C₆ à C₁₂ aryle, C₃ à C₁₂ hétéroaryle, C₁ à C₁₆ alkyle, C₁ à C₁₆ alcoxy, C₁ à C₁₆ alkyle partiellement ou perfluoré, C₁ à C₁₆ alcoxy partiellement ou perfluoré, C₁ à C₁₆ alkyle partiellement ou perdeutéré, C₁ à C₁₆ alcoxy partiellement ou perdeutéré ;
et X¹ étant choisi parmi O, S ou Se, de préférence O et le composé de formule (I) n'incluant pas les composés suivants :

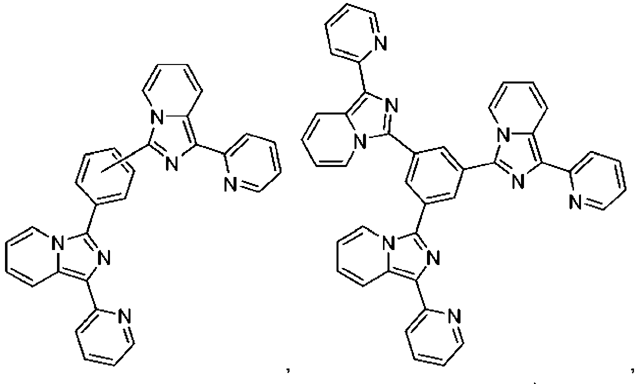

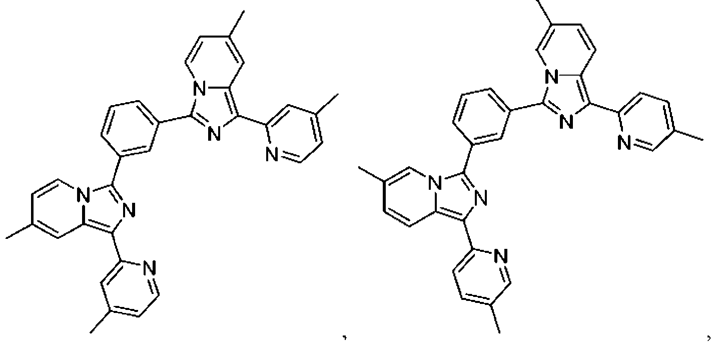

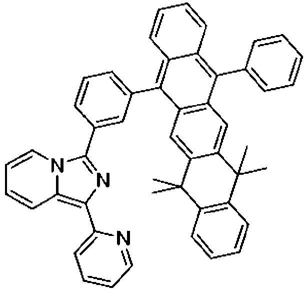

2. Dispositif électronique organique selon la revendication 1, les substituants des autres R¹ à R⁵ et R⁶ à R⁹ qui ne forment pas une simple liaison à la position 3 du groupement 2-azaindolizine étant indépendamment choisis parmi D, -CH=, C₆ à C₁₈ aryle, C₃ à C₂₀ hétéroaryle, C₁ à C₁₆ alkyle, C₁ à C₁₆ alcoxy, C₃ à C₁₆ alkyle ramifié, C₃ à C₁₆ alkyle cyclique, C₃ à C₁₆ alcoxy ramifié, C₃ à C₁₆ alcoxy cyclique, C₁ à C₁₆ alkyle partiellement ou perfluoré, C₁ à C₁₆ alcoxy partiellement ou perfluoré, C₁ à C₁₆ alkyle partiellement ou perdeutéré, C₁ à C₁₆ alcoxy partiellement ou perdeutéré, PX¹(R¹⁰)₂, F ou CN.

3. Dispositif électronique organique selon la revendication 1 ou 2, le composé ayant la formule suivante (1a) :

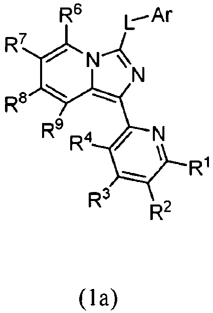

4. Dispositif électronique organique selon l'une quelconque des revendications 1 à 3, la couche organique et/ou le composé de formule (1) ou (1a) étant non émissifs.

5. Dispositif électronique organique selon l'une quelconque des revendications 1 à 4, les R¹ à R⁹ qui ne forment pas une simple liaison à la position 3 du groupement 2-azaindolizine étant indépendamment choisis parmi H, -CH=, C₁ à C₄ alkyle, F ou CN.

6. Dispositif électronique organique selon l'une quelconque des revendications 1 à 5, le composé de formule (1) étant choisi parmi l'une des formules suivantes (2a) à (2f) : 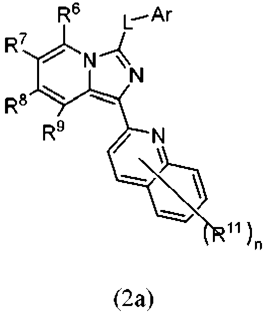 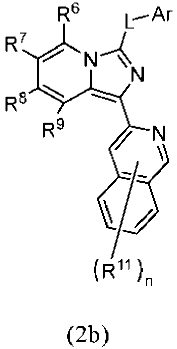 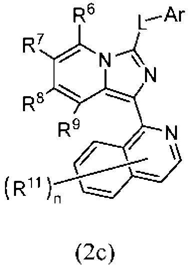 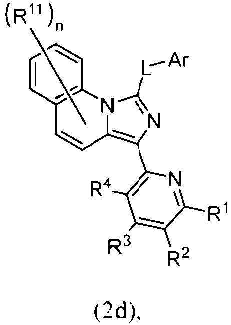 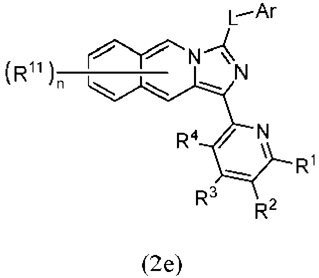 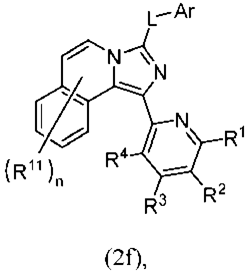 R¹¹ étant indépendamment choisi parmi D, C₆ à C₁₈ aryle, C₃ à C₂₀ hétéroaryle, C₁ à C₁₆ alkyle, C₁ à C₁₆ alcoxy, C₃ à C₁₆ alkyle ramifié, C₃ à C₁₆ alkyle cyclique, C₃ à C₁₆ alcoxy ramifié, C₃ à C₁₆ alcoxy cyclique, C₁ à C₁₆ alkyle partiellement ou perfluoré, C₁ à C₁₆ alcoxy partiellement ou perfluoré, C₁ à C₁₆ alkyle partiellement ou perdeutéré, C₁ à C₁₆ alcoxy partiellement ou perdeutéré, PX¹(R¹⁰)₂, F ou CN ; et
n étant un entier de 0 à 4.

7. Dispositif électronique organique selon l'une quelconque des revendications 1 à 6, le groupement L étant choisi parmi l'un quelconque des groupements suivants E1 à E26 : 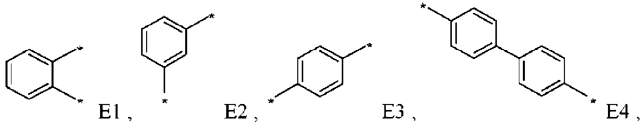 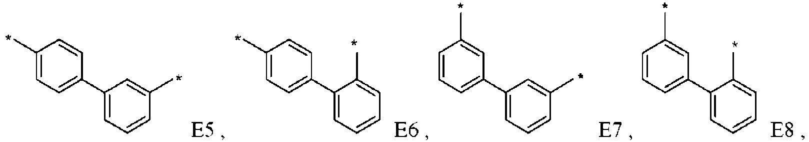 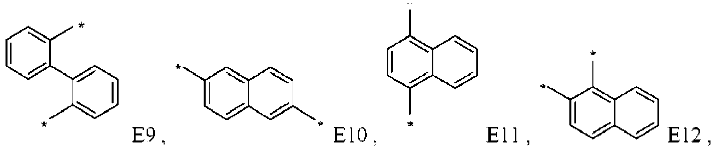 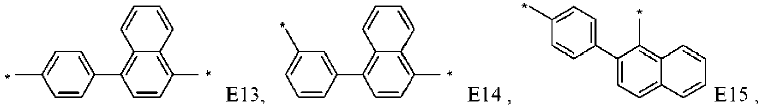 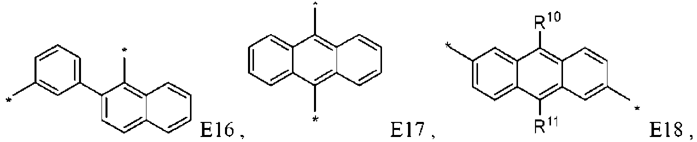 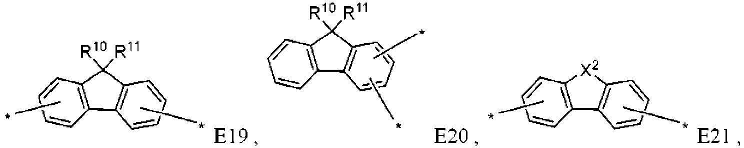 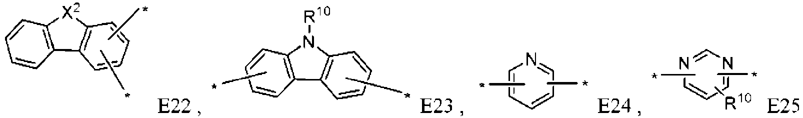 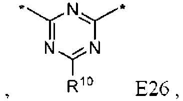 X² étant choisi parmi O ou S, de préférence O ;
R¹⁰ et R¹¹ étant indépendamment choisis parmi H, C₁ à C₁₆ alkyle, C₁ à C₁₆ alcoxy, C₆ à C₁₈ aryle, C₃ à C₂₀ hétéroaryle, C₁ à C₁₆ alkyle perfluoré, C₁ à C₁₆ alcoxy perfluoré.

8. Dispositif électronique organique selon l'une quelconque des revendications 1 à 7, la couche de semi-conducteur organique comprenant un dopant n redox.

9. Dispositif électronique organique selon l'une quelconque des revendications 1 à 8, la couche de semi-conducteur organique comprenant un métal.

10. Dispositif électronique organique selon l'une quelconque des revendications 1 à 9, la couche de semi-conducteur organique comprenant un métal choisi parmi les métaux alcalins, les métaux alcalino-terreux, les métaux des terres rares et les métaux de la première période de transition Ti, V, Cr et Mn.

11. Dispositif électronique organique selon l'une quelconque des revendications 1 à 10, l'au moins une couche photoactive étant une couche émettrice de lumière.

12. Dispositif électronique organique selon l'une quelconque des revendications 1 à 11, le dispositif électronique organique étant un dispositif électroluminescent, de préférence une diode émettrice de lumière organique.

13. Dispositif d'affichage comprenant un dispositif électronique organique selon l'une quelconque des revendications 1 à 12.

14. Composé de formule (1) tel que défini à la revendication 1.
